# EUROPÄISCHE PATENTSCHRIFT

(11) **EP 2 841 030 B1**
(45) Veröffentlichungstag und Bekanntmachung des Hinweises auf die Patenterteilung: **28.09.2016**
(21) Anmeldenummer: 13719829.7
(22) Anmeldetag: 26.04.2013
(51) Int. Cl.: A61F 5/01

(54) **DAUMENORTHESE**
THUMB ORTHOSIS
ORTHÈSE DU POUCE

(30) Priorität: 26.04.2012 DE 102012008565; 26.04.2012 US 201261638746 P
(43) Veröffentlichungstag der Anmeldung: 04.03.2015
(73) Patentinhaber: BSN medical GmbH, 20253 Hamburg (DE)
(72) Erfinder: GRUNDEN, Jennifer, 22769 Hamburg (DE); SCHMELTZPFENNING, Timo, 21244 Buchholz (DE); BAUER, Joachim, 22589 Hamburg (DE)
(74) Vertreter: Uexküll & Stolberg
(86) Internationale Anmeldenummer: PCT/EP2013/058818
(87) Internationale Veröffentlichungsnummer: WO 2013/160478

(56) Entgegenhaltungen:
- EP-A2- 0 850 573
- WO-A1-96/27349
- DE-A1- 3 519 493
- US-A- 4 854 310
- US-B1- 6 520 925

## Beschreibung

Die vorliegende Erfindung betrifft eine Orthese zur Befestigung an einer menschlichen Hand zur Immobilisierung von Daumensattel- und -grundgelenk des Daumens der Hand.

Derartige Orthesen werden dazu verwendet, das Daumensattelgelenk und/oder Daumengrundgelenk eines Benutzers im Rahmen einer Therapie chronischer, posttraumatischer oder postoperativer Reizzustände oder anderer pathologischer Zustände im Bereich dieser Gelenke zu immobilisieren bzw. ruhig zu stellen und in einer festgelegten Stellung zu fixieren. Ein Beispiel für einen pathologischen Zustand, bei dem eine solche Orthese vorteilhaft zum Einsatz kommen kann, ist die sog. Rhizarthrose des Daumens, d.h. Arthrose bzw. degenerativer Knorpelschwund im Bereich des Daumensattelgelenks. Ein weiteres Beispiel ist der sog. Skidaumen, bei dem es nach einem Wegknicken des Daumens im Grundgelenk nach außen, zum Beispiel bei einem Sturz, zu einer Seitenbandruptur des Daumens gekommen ist.

Dabei ist es wünschenswert, dass die Funktionsfähigkeit der Hand des Benutzers möglichst nur insoweit eingeschränkt wird, wie es für die Immobilisierung bzw. Ruhigstellung der betroffenen Gelenke erforderlich ist. Insbesondere ist es wünschenswert, den Benutzer in die Lage zu versetzen, immer noch den Pinzettengriff ausführen zu können, indem der Daumen in Gegenüberstellung mit dem Zeigefinger fixiert ist und der Zeigefinger zum Ergreifen von Gegenständen pinzettenartig in Anlage an den Daumen gebracht werden kann.

Bekannte Orthesen sind im Allgemeinen so ausgestaltet, dass sie bei bestimmungsgemäßem Einsatz an das Handgelenk angrenzen bzw. an diesem enden und daher Bewegungen im Handgelenk stören und Druckstellen provozieren. Ferner sind sie im Allgemeinen so ausgestaltet, dass sie das Daumengrundgelenk umgreifen und eine großflächige Ummantelung des Daumens bis zum Endglied bereitstellen. Ein Beispiel ist die in DE 20 2009 017 438 U1 offenbarte Orthese, die einen schlauchförmigen Hauptschnitt und einen Daumenabschnitt aufweist. Der Hauptabschnitt ist so ausgestaltet und dimensioniert, dass er bei an eine Hand angelegter Orthese den Unterarm zumindest teilweise und den Handwurzelknochenbereich umgibt und sich dabei von einem unterarmseitigen Ende, das zum Ellenbogengelenk weist, über das Handgelenk selbst und die Handwurzelknochen bis zu den Mittelhandknochen der Finger erstreckt. Der Daumenabschnitt erstreckt sich zur Aufnahme des Daumens von dem Hauptabschnitt zur Seite und ist so angeordnet und dimensioniert, dass er im angelegten Zustand den Mittelhandknochen des Daumens umgibt und sich über das Daumengrundgelenk hinaus zumindest entlang eines Teils des Fingergrundgliedes des Daumens erstreckt. Im Bereich des Daumenabschnitts sind Stabilisierungsstäbe eingearbeitet, die sich im angelegten Zustand seitlich in Längsrichtung des Daumens erstrecken und durch den Hauptabschnitt und den Daumenabschnitt fixiert werden.

Orthesen von diesem Aufbau können zwar im Prinzip eine gute Immobilisierungswirkung bereitstellen. In der Praxis ist die tatsächlich erzielte Immobilisierung jedoch unzureichend. So ermöglicht die seitliche Schienung für den Daumen noch eine hohe Restbeweglichkeit. Dennoch schränken die Orthesen in jedem Fall die Beweglichkeit des Handgelenks ein. Außerdem weisen sie häufig hohe textile Anteile auf, die mit den Problemen einer schnellen Verschmutzung und einer dadurch erforderlichen Reinigung verbunden sind, während derer der Patient die Orthese nicht verwenden kann. Die Verwendung von Kunststoffen birgt dagegen bei den bekannten Orthesen ein hohes Druckstellenpotential. Außerdem sind sie häufig schwierig anzulegen, insbesondere unter Berücksichtigung des Umstands, dass der Benutzer nur eine Hand zur Verfügung hat.

Aus der U.S. 4,854,310 ist eine Orthese zur Immobilisierung von Daumensattel- und -grundgelenk bekannt, die einen Mittelhand¬abschnitt aufweist, der einen am Handrücken anliegenden Abschnitt und zwei Endabschnitte hat, die sich an die Handinnenfläche anlegen. Darüber hinaus ist ein Daumenabschnitt vorgesehen, der sich von dem Mittelhandabschnitt beabstandet von einem Ende davon weg erstreckt.

Die U.S. 6, 520, 925, von der die vorliegende Erfindung ausgeht, beschreibt eine Daumenorthese, die einen Mittelhandabschnitt aufweist, der an der Handinnenfläche des Benut-zers anliegt, wobei sich ein Daumenabschnitt von einem Ende des Mittelhandabschnitts weg erstreckt. Ferner ist eine Verschlusseinrichtung zur Befestigung der Orthese an der Hand eines Benutzers vorgesehen.

Es ist Aufgabe der vorliegenden Erfindung, eine einfach aufgebaute Orthese zur Befestigung an einer menschlichen Hand zur Immobilisierung von Daumensattel- und -grundgelenk des Daumens der Hand bereitzustellen, durch die eine zuverlässige Ruhigstellung des Daumens in allen Bewegungsrichtungen erreicht werden kann und die die Beeinträchtigung der Beweglichkeit und Funktionsfähigkeit des Handgelenks gegenüber dem Stand der Technik dennoch verringert und die genannten Nachteile überwindet.

Zur Lösung dieser Aufgabe dienen die Merkmale von Patentanspruch 1. Vorteilhafte Ausführungsformen der Orthese sind Gegenstand der zugehörigen Unteransprüche.

Nach der vorliegenden Erfindung ist vorgesehen, dass eine Orthese zur Befestigung an einer menschlichen Hand zur Immobilisierung von Daumensattel- und -grundgelenk des Daumens der Hand einen steifen Körper aufweist, der, wie noch erläutert werden wird, mehrstückig oder bevorzugt einstückig ausgebildet sein kann. Dabei ist es auch möglich, dass die Orthese insgesamt durch den steifen Körper gebildet wird bzw. aus dem steifen Körper besteht.

Durch seine Steifigkeit ist der Körper in der Lage, die Hand in gewünschter Weise abzustützen, zu stabilisieren und zu immobilisieren, und es wird eine Formstabilität bereitgestellt, so dass der Körper in einfacher Weise mit einer definierten Form an die Hand angelegt werden kann. Trotz der steifen Ausbildung des Körpers wird er im Allgemeinen eine gewisse Elastizität aufweisen, die das Anlegen erleichtert. Ferner ist es bevorzugt, wenn der Körper bei Anwendung einer ausreichenden Kraft, ggf. in Verbindung mit Wärmeeinwirkung, plastisch verformbar ist, um eine einfache Anpassung an die Hände unterschiedlicher Benutzer zu ermöglichen.

Der steife Körper weist zum einen einen Mittelhandabschnitt auf, der durch einen gekrümmten und steifen Abschnitt eines Elements gebildet wird. Dies umfasst auch die Möglichkeit, dass das gesamte Element den Mittelhandabschnitt bildet, und die Möglichkeit, dass der gesamte steife Körper durch das Element gebildet wird bzw. der steife Körper mit dem Element identisch ist. Das Element, der steife Körper bzw. zumindest der Mittelhandabschnitt sind bevorzugt flach, bandförmig oder flächig. Der Mittelhandabschnitt hat ein erstes Ende und ein von diesem beabstandetes zweites Ende. Dabei kann der Mittelhandabschnitt länglich sein, wobei dann das erste und das zweite Ende in Längsrichtung voneinander beabstandet sind.

Der Mittelhandabschnitt ist ferner in der Weise ausgebildet, dass er mit einer menschlichen Hand in Eingriff gebracht werden kann, indem er unterhalb der Fingergrundgelenke von Zeigefinger Mittelfinger, Ringfinger und kleinem Finger und oberhalb der Handwurzel so an die Handinnenfläche der Hand angelegt wird, dass er sich in Umfangsrichtung der Mittelhand zumindest teilweise über die Handinnenfläche erstreckt und dabei vollständig oder teilweise an dieser anliegt. Genauer erstreckt er sich in Umfangsrichtung der Mittelhand bzw. in einer Richtung zwischen den beiden Handkanten über die gesamte Handinnenfläche oder zumindest einen Teil der Handinnenfläche und liegt vollständig oder teilweise an dieser an. Zu diesem Zweck weist sie bevorzugt eine Anlagefläche auf, die bei flacher, bandförmiger bzw. flächiger Ausgestaltung des Mittelhandabschnitts durch eine ausgedehnte Seite des Mittelhandabschnitts gebildet wird. In einer bevorzugten Ausführungsform erstreckt sich der Mittelhandabschnitt in der anatomischen Normalstellung von Hand und Unterarm im Wesentlichen senkrecht zur Längsachse des Unterarms über die Handinnenfläche. Durch diese Ausgestaltung des Mittelhandabschnitts ist dieser im angelegten Zustand in vorteilhafter Weise im Bereich der Mittelhand angeordnet und vom Handgelenk in Richtung auf die Finger beabstandet. Somit wird das Handgelenk in vorteilhafter Weise nicht abgedeckt und bleibt frei beweglich.

Dabei kann vorgesehen sein, dass die beiden Enden des Mittelhandabschnitts durch ein elastisches bzw. flexibles Element oder mehrere elastische bzw. flexible Elemente verbunden sind oder verbunden werden können, das bzw. die zusammen mit dem Mittelhandabschnitt und ggf. weiteren Abschnitten oder Elementen der Orthese einen ringförmig geschlossenen Abschnitt ausbildet bzw. ausbilden, der bei der beschriebenen bestimmungsgemäßen Verwendung die Mittelhand des Benutzers vollständig umläuft.

Außerdem weist der steife Körper einen steifen Daumenabschnitt auf, der in einer bevorzugten Ausführungsform gleichfalls durch einen gekrümmten und steifen Abschnitt des den Mittelhandabschnitt bildenden Elements gebildet wird. Der Daumenabschnitt stellt einen den Daumen aufnehmenden Ansatz an dem Mittelhandabschnitt dar, der sich von dem Mittelhandabschnitt erstreckt und ein erstes Ende, das an dem Mittelhandabschnitt befestigt ist oder durch einen Teil des Mittelhandabschnitts gebildet wird bzw. mit diesem identisch ist, und ein zweites Ende hat. Wenn das erste Ende des Daumenabschnitts durch einen Teil des Mittelhandabschnitts gebildet wird bzw. mit diesem identisch ist, sind der Mittelhandabschnitt und der Daumenabschnitt und damit der steife Körper einstückig ausgebildet. Andernfalls sind der Mittelhandabschnitt und der Daumenabschnitt als separate Komponenten vorgesehen, die starr miteinander verbunden werden können. Im Bereich seines zweiten Endes weist der Daumenabschnitt einen ringförmigen oder ringsegmentförmigen Daumenhalteabschnitt auf.

Insgesamt ist der Daumenabschnitt in der Weise ausgebildet, dass er bei mit der Hand in obiger Weise in Eingriff gebrachtem Mittelhandabschnitt so an dem Daumen der Hand angelegt werden kann, dass sich der Daumen durch den ringförmigen bzw. ringsegmentförmigen Daumenhalteabschnitt erstreckt, d.h. durch die von dem Daumenhalteabschnitt definierte Ringöffnung, und dass sich dann der Daumenhalteabschnitt über zumindest einen Teil des Umfangs des Daumens erstreckt und ihn ganz oder zumindest teilweise umfasst und dabei den Daumen gegen eine Bewegung in Richtung auf die Handfläche, d.h. Adduktion, und gegen eine Bewegung in Gegenrichtung, d.h. Abduktion, sowie gegen eine Flexion des Metacarpalgelenks abstützt. Bevorzugt ist dazu eine konkave, zur Anlage am Daumen vorgesehene Fläche des ringförmigen bzw. ringsegmentförmigen Daumenhalteabschnitts so angeordnet, dass sie bei an einer Hand angelegter Orthese zumindest auch an der zur Handinnenfläche weisenden Innenseite des Daumens anliegt. Es ist aber auch möglich, dass eine solche konkave, zur Anlage am Daumen vorgesehene Fläche zumindest auch auf der gegenüberliegenden Seite des Daumens anliegt. Die Abstützung durch den Daumenhalteabschnitt erfolgt in einer Position, in der der Daumen und der Zeigefinger der Hand gegenübergestellt sind und der Zeigefinger in Richtung auf den Daumen in Anlage an diesen und von dem Daumen weg bewegt werden kann. Mit anderen Worten wird die Hand in einer Funktionsstellung immobilisiert bzw. ruhiggestellt, in der der Benutzer den wichtigen Pinzettengriff ausführen kann.

In einer bevorzugten Ausführungsform sind das Element und dessen Abschnitt, der den Mittelhandabschnitt des steifen Körpers bildet, länglich. Dies umfasst auch die Möglichkeit, dass das gesamte längliche Element den Mittelhandabschnitt bildet. Das erste Ende des Mittelhandabschnitts wird durch eines der beiden Längsenden des länglichen Elements gebildet, und der Mittelhandabschnitt weist das erste Ende und das zweite Ende in der Längsrichtung des Mittelhandabschnitts auf, d.h. der Mittelhandabschnitt weist in Längsrichtung das erste und das zweite Ende auf. Der Mittelhandabschnitt ist in der Weise gekrümmt, dass er die Form eines Segments eines - nicht kreisförmigen - Rings hat, der eine längliche und ovale bzw. im Wesentlichen ovale Ringöffnung umschließt und definiert. Daher sind das erste und das zweite Ende des Mittelhandabschnitts - wie bereits angegeben - voneinander beabstandet bzw. liegen einander auf Abstand gegenüber. Mit anderen Worten ist der Mittelhandabschnitt in der Form eines offenen Rings vorgesehen.

Der Mittelhandabschnitt ist dann ferner in der Weise ausgebildet, dass er mit einer menschlichen Hand in Eingriff gebracht werden kann, indem er nicht nur in der beschriebenen Weise an die Handinnenfläche der Hand gelegt wird, sondern gleichzeitig unterhalb der Fingergrundgelenke von Zeigefinger Mittelfinger, Ringfinger und kleinem Finger (und oberhalb oder bevorzugt unterhalb des Daumengrundgelenks) und - aufgrund der länglichen Ausbildung - oberhalb der Handwurzel so um den Handrücken und die Handinnenfläche der Hand gelegt wird, dass er sich in Umfangsrichtung der Mittelhand um diese erstreckt und dabei vollständig oder teilweise an dieser anliegt und die Mittelhand teilweise umgreift. Genauer erstreckt er sich in Umfangsrichtung der Mittelhand bzw. in einer Richtung zwischen den beiden Handkanten vollständig oder teilweise über den Handrücken und liegt dabei vollständig oder teilweise an diesem an. Ferner verläuft er um eine oder beide Handkanten und liegt vollständig oder teilweise an einer Handkante oder an beiden Handkanten an. Schließlich erstreckt er sich in Umfangsrichtung der Mittelhand bzw. in einer Richtung zwischen den beiden Handkanten über die gesamte Handinnenfläche oder zumindest einen Teil der Handinnenfläche und liegt vollständig oder teilweise an dieser an. Es ist dabei bevorzugt, wenn der Mittelhandabschnitt die Mittelhand in einer Ebene umschließt, die in der anatomischen Normalstellung von Hand und Unterarm im Wesentlichen senkrecht zur Längsachse des Unterarms verläuft. Durch diese Ausgestaltung des Mittelhandabschnitts sind die beiden Längskanten des Mittelhandabschnitts im angelegten Zustand in vorteilhafter Weise im Bereich der Mittelhand angeordnet und vom Handgelenk in Richtung auf die Finger beabstandet. Somit wird das Handgelenk in vorteilhafter Weise nicht abgedeckt und bleibt frei beweglich.

Außerdem ist es in dieser Ausführungsform bevorzugt, wenn der Daumenabschnitt länglich ist und sein erstes und zweites Ende in seiner Längsrichtung aufweist.

In einer bevorzugten Ausführungsform ist der steife Körper insgesamt aus dem Element bzw. länglichen Element ausgebildet, und der Daumenabschnitt ist - wie der Mittelhandabschnitt - ein - bevorzugt länglicher - (Teil-) Abschnitt des Elements bzw. länglichen Elements. Das erste Ende des Mittelhandabschnitts wird dann durch ein Ende bzw. eines der beiden Längsenden des steifen Körpers gebildet, und das zweite Ende des Daumenabschnitts entspricht dem gegenüberliegenden Ende des steifen Körpers. Der Daumenabschnitt erstreckt sich von dem zweiten Ende des Mittelhandabschnitts, und das erste Ende des Daumenabschnitts ist dazu an dem ersten Ende des Mittelhandabschnitts befestigt (bzw. kann an diesem befestigt werden) oder wird bevorzugt durch das zweite Ende des Mittelhandabschnitts gebildet bzw. ist mit diesem identisch. In letzterem Fall sind der steife Körper und das Element bzw. das längliche Element jeweils einstückig ausgebildet.

Diese beiden Ausgestaltungen einer Orthese haben den Vorteil, dass mit ihnen eine zuverlässige Ruhigstellung erreicht werden kann, die die Funktionsfähigkeit der Hand wenig beeinträchtigt und insbesondere die Beweglichkeit des Handgelenks nicht einschränkt. Wenn der Mittelhandabschnitt so ausgestaltet ist, dass er in der oben beschriebenen Weise bei an die Hand angelegter Orthese auch über den Handrücken verläuft, stabilisiert im Einsatz dieser über den Handrücken verlaufende Teil des Mittelhandabschnitts in vorteilhafter Weise die Orthese zuverlässig bei dem Versuch einer Bewegung des Daumens gegenüber dem Daumenabschnitt. Dasselbe kann alternativ auch durch einen Mittelhandabschnitt erreicht werden, der so ausgestaltet ist, dass er bei an die Hand angelegter Orthese nicht über den Handrücken verläuft bzw. an diesem anliegt, wenn der Mittelhandabschnitt durch eine geeignete Verschluss- bzw. Befestigungseinrichtung zuverlässig in Anlage an der Handinnenfläche gehalten wird. Wie bereits erwähnt worden ist und wie weiter unten noch näher erläutert wird, kann eine solche Befestigungseinrichtung zum Beispiel ein oder mehrere flexible oder elastische Bänder aufweisen, die zusammen mit dem Mittelhandabschnitt und ggf. weiteren Abschnitten der Orthese im angelegten Zustand die Hand umschließen, wobei die Bänder zumindest teilweise über den Handrücken verlaufen. Die obigen Vorteile werden durch die Ausbildung von Mittelhandabschnitt und Daumenabschnitt in Form von Teilen bzw. Abschnitten eines steifen Körpers besonders einfach realisiert. Es kann eine Ruhigstellung des Daumens in allen Bewegungsrichtungen erreicht werden, d.h. in Bezug auf Rotation, Ab- und Adduktion sowie Dorsal- und Palmarflexion. Pinzettengriff, volle Beweglichkeit des Handgelenks und einfach anzulegen.

Außerdem kann die Orthese aufgrund der Ausbildung von Mittelhandabschnitt und Daumenabschnitt durch einen einzigen steifen Körper einfach mit einer Hand angelegt werden. Es ist lediglich erforderlich, den Mittelhandabschnitt um die Mittelhand anzuordnen und anschließend oder dabei den Daumen an dem Daumenabschnitt anzuordnen und festzulegen. Der Daumenabschnitt wird bei der Befestigung des Mittelhandabschnitts an der Mittelhand automatisch in der richtigen Position angeordnet. Ferner wird im Vergleich zum Stand der Technik trotz wenig Anlagefläche ein höherer Immobilisierungsgrad erreicht.

In einer bevorzugten Ausführungsform ist in dem Bereich des erstes Endes des Daumenabschnitts und insbesondere in dem Übergangsbereich zwischen Mittelhandabschnitt und Daumenabschnitt eine Versteifung vorgesehen. Wenn sich der Daumenabschnitt von dem zweiten Ende des Mittelhandabschnitts erstreckt, ist eine solche Versteifung bevorzugt, in dem Bereich des ersten Endes des Daumenabschnitts und des zweiten Endes des Mittelhandabschnitts vorgesehen. Dadurch wird der Übergangsbereich von Mittelhandabschnitt und Daumenabschnitt verstärkt, so dass der Daumenabschnitt während der Benutzung zuverlässig und stabil in der richtigen Position in Bezug auf den Mittelhandabschnitt gehalten wird. Alternativ oder bevorzugt zusätzlich kann auch an einer oder mehreren anderen Stellen des steifen Körpers, ggf. punktuell, eine Verstärkung mittels geeigneter Profilierung oder dem Vorsehen geeigneter Versteifungsmaterialien vorgesehen sein. Da der Körper steif ist, können solche zusätzlichen Versteifungen bzw. Verstärkungen vorgesehen werden, ohne dass die Anwendung der Orthese durch den Benutzer und insbesondere ihr Anlegen beeinträchtigen wird und dass sich für den Benutzer während des Tragens Nachteile ergeben.

In einer bevorzugten Ausführungsform ist ein Teilbereich des steifen Körpers oder der gesamte steife Körper zur besseren individuellen Anpassung an die Hände verschiedener Benutzer plastisch verformbar. Beispielsweise kann der steife Körper zumindest in Teilbereichen aus einem oder mehreren Materialien ausgebildet sein, die eine solche Verformung nach Erwärmung auf eine bestimmte Temperatur zulassen und nach der Abkühlung wieder eine ausreichende Steifigkeit und Formstabilität gewährleisten.

In einer bevorzugten Ausführungsform weisen das Element bzw. längliche Element des steifen Körpers und/oder der Daumenabschnitt ein oder mehrere Metalle, insbesondere Aluminium, und/oder ein oder mehrere Kunststoffmaterialien auf. Wird Metall verwendet, kann in vorteilhafter Weise insbesondere Aluminium gewählt werden, und als Kunststoff kann in vorteilhafter Weise ein thermoplastisches Elastomer eingesetzt werden. In einer besonders bevorzugten Ausgestaltung weisen das Element bzw. längliche Element und/oder der Daumenabschnitt einen Metallkern auf, der von einem Kunststoffmaterial ummantelt ist. Der Metallkern kann dazu insbesondere mit dem Kunststoffmaterial umspritzt werden.

In einer bevorzugten Ausgestaltung von Ausführungsformen, in denen der Mittelhandabschnitt die Form eines Segments eines eine längliche Ringöffnung umschließenden Rings hat, ist der Mittelhandabschnitt so dimensioniert, dass er sich über mindestens 30%, bevorzugt mindestens 40% und mehr bevorzugt mindestens 50% des Umfangs der Ringöffnung erstreckt, die durch den dem Segment entsprechenden Ring definiert wird. Auf diese Weise kann ein sicheres Umgreifen der Mittelhand und zumindest eine gute Vorfixierung der Orthese an der Hand erreicht werden.

Es ist bevorzugt, dass der steife Körper eine Polsterung aufweist, die beispielsweise durch textile Polster, Schaumstoff, Gel und/oder Luft bereitgestellt werden kann. Die Polsterung ist so angeordnet, dass sie nach Befestigung an der Hand eines Benutzers mit der Hand in Anlage kommt. Die Polsterung kann auch so ausgestaltet sein, dass sie nicht nur den Tragekomfort für den Benutzer steigern, sondern dass mit ihr auch in einem gewissen Maß Größenunterschiede verschiedener Hände ausgeglichen werden können.

In einer bevorzugten Ausgestaltung von Ausführungsformen, in denen der Mittelhandabschnitt die Form eines Segments eines eine längliche Ringöffnung umschließenden Rings hat, ist der Mittelhandabschnitt in der Weise ausgestaltet, dass sein erstes und zweites Ende sich auf einer langen Seite der länglichen Ringöffnung gegenüberliegen, die durch den gedachten, nicht kreisförmigen, länglichen Ring definiert wird, von dem das den Mittelhandabschnitt darstellende Segment ein Teil ist. Der zwischen den beiden Enden vorhandene Zwischenraum ist dabei so angeordnet, dass er sich bei an einer Hand angelegter Orthese im Bereich der Handinnenfläche befindet. Daher erstreckt sich der Mittelhandabschnitt über den gesamten Handrücken und umgreift beiden Handkanten. Es ist dabei von Vorteil, wenn das zweite Ende des Mittelhandabschnitts im angelegten Zustand der Orthese im Bereich des Daumens angeordnet ist, um den Daumenabschnitt möglichst kurz und damit stabil halten zu können.

In dieser Ausführungsform ist der Daumenabschnitt ferner in Bezug auf den Verlauf des Mittelhandabschnitts in dem Bereich, in dem sich der Daumenabschnitt von dem Mittelhandabschnitt weg erstreckt, abknickend angeordnet, z.B. in Bezug auf den Verlauf des Mittelhandabschnitts an dessen zweitem Ende, wenn sich der Daumenabschnitt von dem zweiten Ende des Mittelhandabschnitts erstreckt. Bevorzugt erstreckt sich der Daumenabschnitt im Bereich seines ersten Endes im Wesentlichen senkrecht zum jeweiligen Bereich des Mittelhandabschnitts.

Bei dieser Ausführungsform ist es ferner bevorzugt, wenn der Daumenhalteabschnitt ringsegmentförmig ausgebildet ist und bevorzugt mehr als 50% des Umfangs der Ringöffnung umfasst, die durch den dem ringsegmentförmigen Daumenhalteabschnitt entsprechenden Ring definiert wird, bevorzugt mehr als 60%, mehr bevorzugt mehr als 70% und am meisten bevorzugt mehr als 80%. Dadurch umgreift er im angelegten Zustand der Orthese mehr als 50%, bevorzugt mehr als 60%, mehr bevorzugt mehr als 70% und am meisten bevorzugt mehr als 80% des Umfangs des Daumens und verhindert auf diese Weise ein absichtliches seitliches Herausbewegen oder zumindest ein seitliches Herausrutschen des Daumens. In einer besonders vorteilhaften Ausgestaltung ist der Daumenhalteabschnitt so weit geschlossen ausgebildet, dass der Daumen nicht mehr seitlich, sondern nur noch axial in den Daumenhalteabschnitt eingeführt werden kann.

Bei dieser Ausführungsform ist es außerdem bevorzugt, wenn der Mittelhandabschnitt in einer ersten Ebene verläuft und der ringsegmentförmige Daumenhalteabschnitt in einer zweiten Ebene verläuft, wobei die erste und zweite Ebene gegeneinander verkippt sind.

Bei dieser Ausführungsform ist es außerdem bevorzugt, wenn der Mittelhandabschnitt in der Nähe seines ersten Endes eine Auswölbung aufweist, die zur Anlage an die Mulde in der Handinnenfläche einer menschlichen Hand und die zu diesem Zweck nach innen in die längliche Ringöffnung vorsteht, die durch den gedachten, nicht kreisförmigen, länglichen Ring definiert wird, von dem das den Mittelhandabschnitt darstellende Segment ein Teil ist. Mit anderen Worten ist die Auswölbung ein Teil der palmaren Fläche des Mittelhandabschnitts in der Nähe von dessen erstem Ende, das bei an eine Hand angelegter Orthese im Bereich der Handinnenfläche angeordnet ist.

Bei dieser Ausführungsform ist es außerdem bevorzugt, wenn der Mittelhandabschnitt so ausgebildet ist, dass der Abstand zwischen seiner am Handrücken zur Anlage kommenden Fläche und seiner an der Handinnenfläche zur Anlage kommenden Fläche in Richtung auf diejenige Längskante des Mittelhandabschnitts zunimmt, die bei angelegter Orthese in Richtung auf die Handwurzel weist. Dies berücksichtigt die Form menschlicher Hände, die an den beiden Handkanten unterhalb des kleinen Fingers und des Zeigefingers jeweils eine Auswölbung aufweisen, und die Form des unteren Daumenbereichs. Auf diese Weise ist es möglich, einen festen Sitz alleine durch den steifen Körper ohne zusätzliche Verschlusselemente zu gewährleisten.

Bei dieser Ausführungsform ist es außerdem bevorzugt, wenn das längliche Element an dem ersten Ende des Mittelhandabschnitts oder zumindest in der Nähe des ersten Endes des Mittelhandabschnitts die größte Breite - gemessen zwischen den beiden Längskanten - aufweist und sich in Richtung auf das gegenüberliegende Ende des länglichen Elements bzw. das zweite Ende des Mittelhandabschnitts oder das zweite Ende des Daumenabschnitts verjüngt. Dabei kann sich die Breite insbesondere zwischen dem ersten und dem zweiten Ende kontinuierlich verringern. Auch diese Ausgestaltung trägt zu einem festen Sitz der Orthese bei, da sie verhindert, dass der Patient bei dem Versuch, seinen Daumen in Adduktion zu bringen, den Mittelhandabschnitt von der Hand weg und die Hand dadurch aus dem Mittelhandabschnitt herausdrückt.

In einer alternativen bevorzugten Ausgestaltung von Ausführungsformen, in denen der Mittelhandabschnitt die Form eines Segments eines eine längliche Ringöffnung umschließenden Rings hat, ist der Mittelhandabschnitt in der Weise ausgestaltet, dass sein erstes und zweites Ende sich in der Weise gegenüberliegen, dass sich zumindest ein Teil des zwischen ihnen gebildete Zwischenraums auf einer schmalen Seite der Ringöffnung befindet, die durch den dem Segment entsprechenden Ring definiert wird. Dieser Teil des Zwischenraums ist außerdem im an eine Hand angelegten Zustand im Bereich des Daumens angeordnet. Es ist dabei bevorzugt, wenn sich mindestens ein Ende des Mittelhandabschnitts bei an eine Hand angelegter Orthese im Bereich des Daumens befindet. Beispielsweise kann der Mittelhandabschnitt in der Weise ausgestaltet sein, dass sein erstes und zweites Ende sich auf einer schmalen Seite der länglichen Ringöffnung gegenüberliegen, die durch den gedachten, nicht kreisförmigen, länglichen Ring definiert wird, von dem das den Mittelhandabschnitt darstellende Segment ein Teil ist. Der zwischen den beiden Enden vorhandene Zwischenraum ist dann so angeordnet und dimensioniert, dass sich der Zwischenraum und das erste und zweite Ende des Mittelhandabschnitts bei an eine Hand angelegter Orthese im Bereich des Daumens und vorteilhaft im Bereich der Handkante befinden. Daher erstreckt sich der Mittelhandabschnitt vollständig oder zumindest teilweise über den Handrücken und umgreift die dem Daumen gegenüberliegende Handkante. Das erste Ende, und bevorzugt auch das zweite Ende, des Mittelhandabschnitts sind im angelegten Zustand im Bereich des Daumensattelgelenks angeordnet. Mit anderen Worten ist der Mittelhandabschnitt so ausgestaltet, dass er sich bei an eine Hand angelegter Orthese von der Handinnenfläche in der Nähe des Daumensattelgelenks von dem Daumen weg um die Handkante herum und über einen Teil des Handrückens oder den Handrücken erstreckt, wo dann das erste Ende angeordnet ist, zum Beispiel bis in die Nähe des Daumensattelgelenks.

In dieser Ausführungsform ist ferner der Daumenabschnitt so angeordnet, dass er sich zumindest im Bereich seines ersten Endes in dieselbe oder im Wesentlichen dieselbe Richtung wie der Mittelhandabschnitt im Bereich von dessen zweitem Ende verläuft und sich von dem Mittelhandabschnitt weg erstreckt. Mit anderen Worten ist der Daumenabschnitt eine kontinuierliche Verlängerung bzw. ein kontinuierlicher Fortsatz des Mittelhandabschnitts und knickt anders als in der vorhergehenden Ausführungsform nicht ab.

In dieser Ausführungsform kann in bevorzugten Ausgestaltungen vorgesehen sein, dass wie oben für die vorhergehende alternative Ausführungsform angegeben, der Daumenhalteabschnitt ringsegmentförmig ausgebildet ist und mehr als 50% des Umfangs der Ringöffnung umfasst, die durch den dem ringsegmentförmigen Daumenhalteabschnitt entsprechenden Ring definiert wird, dass der Mittelhandabschnitt und der ringsegmentförmige Daumenhalteabschnitt in gegeneinander verkippten Ebenen verlaufen, dass der Abstand zwischen der am Handrücken zur Anlage kommenden Fläche des Mittelhandabschnitts und seiner an der Handinnenfläche zur Anlage kommenden Fläche in Richtung auf diejenige Längskante des Mittelhandabschnitts zunimmt, die bei angelegter Orthese in Richtung auf die Handwurzel weist, und/oder dass das längliche Element an dem ersten Ende des Mittelhandabschnitts oder zumindest in der Nähe des ersten Endes des Mittelhandabschnitts die größte Breite - gemessen zwischen den beiden Längskanten - aufweist und sich in Richtung auf das gegenüberliegende Ende des länglichen Elements bzw. das zweite Ende des Mittelhandabschnitts oder das zweite Ende des Daumenabschnitts verjüngt. Diesbezüglich gelten dieselben Details und Vorteile, wie sie oben für die alternative Ausführungsform ausgeführt worden sind.

Außerdem kann auch in dieser Ausführungsform in vorteilhafter Weise vorgesehen sein, dass der Mittelhandabschnitt eine Auswölbung aufweist, die zur Anlage an die Mulde in der Handinnenfläche einer menschlichen Hand und die zu diesem Zweck nach innen in die längliche Ringöffnung vorsteht, die durch den gedachten, nicht kreisförmigen, länglichen Ring definiert wird, von dem das den Mittelhandabschnitt darstellende Segment ein Teil ist. Mit anderen Worten ist die Auswölbung ein Teil der palmaren Fläche des Mittelhandabschnitts, das bei an eine Hand angelegter Orthese im Bereich der Handinnenfläche angeordnet ist.

In dieser Ausführungsform ist es ferner bevorzugt, wenn die Orthese ein Verschlusselement zum Fixieren der Orthese enthält, das ein Band aufweist, das bevorzugt mit einem Klettverschlusselement ausgestattet ist.

Das Band eines solchen Verschlusselements kann bevorzugt mit einem Ende an dem Daumenabschnitt befestigt sein oder werden. Dabei ist es so ausgestaltet und befestigt bzw. kann so befestigt werden, dass es bei an eine Hand angelegter Orthese in der Weise um den im Daumenhalteabschnitt angeordneten Daumen geführt werden kann, dass es den Umfang des Daumenhalteabschnitts ganz oder zumindest teilweise umschließt. Bei ringsegmentförmigem Daumenhalteabschnitt ist das Band dabei bevorzugt so ausgestaltet und befestigt bzw. kann so befestigt werden, dass es bei an eine Hand angelegter Orthese in der Weise um den im Daumenhalteabschnitt angeordneten Daumen geführt werden kann, dass der Zwischenraum zwischen den beiden Längsenden des ringsegmentförmigen Daumenhalteabschnitts überspannt wird und die Kombination aus Daumenhalteabschnitt und Band ringförmig geschlossen ist. Bevorzugt kann das Band dabei in diesem Bereich in Anlage an den Daumen gebracht werden. Es ist in diesem Zusammenhang bevorzugt, wenn das am Daumenabschnitt befestigte Ende des Bandes an der konvexen Fläche des Daumenhalteabschnitt befestigt ist oder werden kann, vorteilhaft in der Weise, dass das Band in der Nähe dieses Endes entlang der konvexen Fläche verläuft und an dieser befestigt ist oder werden kann. Um den Daumen zu umschließen, muss das Band dann nur diesen Verlauf fortsetzend und ggf. über den Zwischenraum zwischen den beiden Längsenden des ringsegmentförmigen Daumenhalteabschnitts geführt werden, der dann von dem Band überspannt wird.

Im Fall der obigen Ausführungsformen mit einem ein Band aufweisenden Verschlusselement ist es ferner bevorzugt, wenn das Band so ausgestaltet und befestigt ist bzw. so befestigt werden kann, dass es im angelegten Zustand der Orthese vollständig um den Daumen herum und über das Daumengrundgelenk und die dorsale Seite des Daumens geführt und mit seinem dem am Daumenabschnitt befestigten Ende gegenüberliegenden Ende an einem Abschnitt des Mittelhandabschnitts befestigt werden kann, der bei an eine Hand angelegter Orthese im Bereich des Handrückens angeordnet ist. Weist das Band ein Klettverschlusselement auf, ist an diesem Abschnitt des Mittelhandabschnitts bevorzugt ein entsprechenden Klettverschlusselement angeordnet.

Erfindungsgemäß ist der Mittelhandabschnitt in der Weise ausgebildet, dass er ausschließlich im Bereich der Handinnenfläche bzw. Handfläche an einer menschlichen Hand anliegt, wenn die Orthese bestimmungsgemäß an die Hand angelegt worden ist. Wie oben bereits erläutert worden ist, ist in diesem angelegten Zustand der Orthese zum einen der Mittelhandabschnitt so mit der Hand in Eingriff gebracht worden, dass er unterhalb der Fingergrundgelenke von Zeigefinger, Mittelfinger, Ringfinger und kleinem Finger an der Handinnenfläche der Hand anliegt und sich in einer Richtung zwischen den beiden Handkanten über zumindest einen Teil der Handinnenfläche erstreckt und zumindest teilweise an dieser anliegt. Zum anderen ist der Daumenabschnitt so an dem Daumen der Hand angelegt worden, dass der Daumen in der durch den Daumenhalteabschnitt definierten Ringöffnung angeordnet ist und der Daumenhalteabschnitt sich über zumindest einen Teil des Umfangs des Daumens erstreckt und ihn zumindest teilweise umfasst.

Mit anderen Worten ist der Mittelhandabschnitt so ausgebildet, dass er sich in dem an die Hand angelegten Zustand nicht über Teile des Handrückens und bevorzugt auch nicht über eine oder beide Handkanten erstreckt.

Ferner weist die Orthese erfindungsgemäß eine Verschluss- bzw. Befestigungseinrichtung zum Befestigen der Orthese in dem angelegten Zustand an der Hand auf. Die Verschlusseinrichtung weist einen oder mehrere erste Befestigungsabschnitte auf, die so angeordnet und ausgestaltet sind, dass sie in dem angelegten Zustand der Orthese derart angeordnet und an dem Mittelhandabschnitt und/oder dem Daumenabschnitt fixiert werden können, dass sie jeweils über den Handrücken der Hand verlaufen und zusammen mit dem Mittelhandabschnitt zumindest einen Teil eines Abschnitts der Orthese bildet, der die Hand ringförmig umschließt. Auf diese Weise kann die Orthese von dem ersten Befestigungsabschnitt oder den ersten Befestigungsabschnitten - entweder alleine oder zusammen mit weiteren Abschnitten der Verschlusseinrichtung - einfach uns sicher an der Hand festgehalten bzw. fixiert werden. Diese Verschluss- bzw. Befestigungseinrichtung oder zumindest die ersten Befestigungsabschnitte oder mindestens einer der ersten Befestigungsabschnitte können entweder als separate Komponenten vorgesehen sein, die vollständig von dem Mittelhandabschnitt und dem Daumenabschnitt bzw. dem Rest der Orthese gelöst und zur Befestigung wieder damit verbunden werden können, oder als Komponenten, die dauerhaft an dem Mittelhandabschnitt, dem Daumenabschnitt und/oder einem anderen Teil der Orthese befestigt sind. In letzterem Fall kann insbesondere vorgesehen sein, dass die betreffenden ersten Befestigungsabschnitte jeweils an einer Stelle dauerhaft befestigt sind und nach Anordnung in der Weise, dass sie über den Handrücken verlaufen, zur Fixierung an einer anderen Stelle lösbar befestigt werden können.

Derartige erste Befestigungsabschnitte können zum Beispiel in vorteilhafter Weise durch jeweils ein flexibles und/oder elastisches Band gebildet werden. Die Bänder können in einer Ausgestaltung jeweils an einem Ende an dem Mittelhandabschnitt oder dem Daumenabschnitt befestigt und angepasst sein, um von diesem Ende beabstandet lösbar mit dem ersten Ende des Mittelhandabschnitts verbunden zu werden. Insbesondere ist jedes dieser Bänder bevorzugt so angeordnet, dass es in dem angelegten Zustand der Orthese ausgehend von seinem an dem Mittelhandabschnitt oder dem Daumenabschnitt befestigten Ende über den Handrücken an diesem anliegend zum ersten Ende des Mittelhandabschnitts geführt werden kann. Zur lösbaren Verbindung mit dem ersten Ende des Mittelhandabschnitts können die Bänder jeweils Klettelemente aufweisen, die mit entsprechenden, geeignet angeordneten anderen Klettelementen an den Bändern selbst oder an dem Mittelhandabschnitt oder einem anderen Teil der Orthese zusammenwirken können. In diesem Zusammenhang können an dem ersten Ende oder einem mit diesem verbundenen Abschnitt eine oder mehrere Öffnungen vorgesehen sein, durch die jeweils ein oder mehrere der Bänder geführt werden können. Nach dem Durchführen, kann jedes Band zum Beispiel an sich selbst befestigt werden, beispielsweise mit Hilfe der oben erwähnten Klettelemente, so dass jeweils ein Schlaufenabschnitt entsteht, der durch eine der Öffnungen verläuft.

Die obige Ausführungsform hat den Vorteil, dass aufgrund der Begrenzung der Größe des steifen Mittelhandabschnitts das Gewicht gering gehalten werden kann und die Orthese besonders angenehm zu tragen ist.

In dieser Ausführungsform ist es ferner bevorzugt, wenn der Daumenhalteabschnitt ringsegmentförmig ausgebildet ist. Dabei kann vorteilhaft vorgesehen sein, dass er mehr als 50% des Umfangs der Ringöffnung umfasst, die durch den dem ringsegmentförmigen Daumenhalteabschnitt entsprechenden Ring definiert wird, bevorzugt mehr als 60%, mehr bevorzugt mehr als 70% und am meisten bevorzugt mehr als 80%. Dadurch umgreift er im angelegten Zustand der Orthese mehr als 50%, bevorzugt mehr als 60%, mehr bevorzugt mehr als 70% und am meisten bevorzugt mehr als 80% des Umfangs des Daumens und verhindert auf diese Weise ein absichtliches seitliches Herausbewegen oder zumindest ein seitliches Herausrutschen des Daumens. In einer besonders vorteilhaften Ausgestaltung ist der Daumenhalteabschnitt so weit geschlossen ausgebildet, dass der Daumen nicht mehr seitlich, sondern nur noch axial in den Daumenhalteabschnitt eingeführt werden kann.

In dieser Ausführungsform und insbesondere bei ringsegmentförmiger Ausgestaltung des Daumenhalteabschnitts ist es außerdem bevorzugt, wenn die Verschlusseinrichtung ferner einen oder mehrere zweite Befestigungsabschnitte aufweist, die jeweils so angeordnet und ausgestaltet sind, dass sie in dem angelegten Zustand der Orthese in der Weise zumindest teilweise um den Daumen geführt werden können, dass sie den Daumen zusammen mit dem Daumenhalteabschnitt ringförmig umschließen. Derartige zweite Befestigungsabschnitte können zum Beispiel in vorteilhafter Weise durch jeweils ein flexibles und/oder elastisches Band gebildet werden. Diese können insbesondere mit einem Ende an dem Daumenabschnitt befestigt sein und beispielsweise ein Klettelement aufweisen, mit dem sie nach Führung um den Daumen - insbesondere unter Verwendung eines entsprechenden, geeignet angeordneten anderen Klettelements - fixiert werden können.

Erfindungsgemäß weist der Mittelhandabschnitt eine erste Fläche zur Anlage an der Handinnenfläche und eine gegenüberliegende zweite Fläche auf, die in dem angelegten Zustand der Orthese von der Hand weg gerichtet ist, wobei die erste Fläche zwischen dem ersten und zweiten Ende des Mittelhandabschnitts zwei konkave Abschnitte und einen dazwischen angeordneten konvexen Abschnitt aufweist. Die erste Fläche stellt dann eine palmare Fläche bzw. Anlagefläche dar. Dadurch ist eine besonders gute Anlage an die Handinnenfläche einer menschlichen Hand und dementsprechend eine besonders gute Anlage und Stützwirkung möglich, insbesondere weil die erste Fläche durch die durch den konvexen Abschnitt bereitgestellte Auswölbung und beidseitig angrenzenden konkaven Abschnitt der anatomischen Form einer Handinnenfläche mit einer Mulde zwischen dem Ende des auslaufenden Daumenballens und dem Abschnitt in der Nähe der gegenüberliegenden Handkante entspricht. Dabei ist es ferner bevorzugt, wenn in jedem der beiden konkaven Abschnitte eine Durchgangsöffnung in der ersten Fläche und durch den Mittelhandabschnitt ausgebildet ist. Dies verbessert den Tragekomfort und senkt das Gewicht der Orthese weiter. Die Durchgangsöffnungen haben bevorzugt radiale Abmessungen, die größer als die radialen Abmessungen der die Durchgangsöffnungen umgebenden Bereiche des Mittelhandabschnitts, des steifen Körpers bzw. der Orthese. Die Durchgangsöffnungen sind bevorzugt zur teilweisen Aufnahme eines Teils des Daumenballens bzw. eines Abschnitt in der Nähe der gegenüberliegenden Handkante angeordnet und angepasst.

Außerdem kann der Mittelhandabschnitt in dieser Ausführungsform im Bereich des konvexen Abschnitts in einer Richtung quer zur Richtung zwischen dem ersten und zweiten Ende des Mittelhandabschnitts und entlang der ersten Fläche eine Verjüngung aufweisen, d.h. senkrecht zu einer die erste und zweite Fläche verbindenden Kante bzw. Randfläche des Mittelhandabschnitts. Mit anderen Worten weist der Mittelhandabschnitt - ggf. zusammen mit einem an das erste Ende des Mittelhandabschnitts angrenzenden Abschnitt der Orthese - bei Draufsicht auf die erste Fläche die Form einer "8" oder eines "B" auf. Es hat sich gezeigt, dass dadurch die Anlage und die Stützwirkung noch weiter verbessert werden können und ein Abheben des Mittelhandabschnitts bei Bewegung der Hand zuverlässig verhindert werden kann.

In dieser Ausführungsform ist es ferner bevorzugt, wenn der Mittelhandabschnitt an seinem ersten Ende in einen flexiblen Abschnitt übergeht, wobei der flexible Abschnitt bevorzugt einstückig mit dem Mittelhandabschnitt ausgebildet ist. Beispielsweise können der flexible Abschnitt und der Mittelhandabschnitt bzw. der flexible Abschnitt und der steife Körper durch eine einzige Komponente bzw. einen einzigen Körper gebildet werden, insbesondere durch eine einzige Komponente bzw. Kunststoffkomponente, in deren Innerem überall oder im Wesentlichen überall mit Ausnahme des flexiblen Abschnitts Versteifungselemente angeordnet sind oder die auf andere Weise überall oder im Wesentlichen überall mit Ausnahme des flexiblen Abschnitts versteift ist. Der flexible Abschnitt erlaubt eine bessere Anpassung an eine menschliche Hand, insbesondere im Bereich der Handkante. Vor allem ist es bevorzugt, wenn die ersten Befestigungsabschnitte - beispielsweise in der oben beschriebenen Ausgestaltung als Bändern - mit dem ersten Ende des Mittelhandabschnitts über den flexiblen Abschnitt verbunden werden können, indem der flexible Abschnitt eine Einrichtung zur lösbaren Befestigung der ersten Befestigungsabschnitte an dem flexiblen Abschnitt aufweist. Diese Einrichtung kann zum Beispiel eine oder mehrere Durchgangsöffnungen aufweisen, durch die die ersten Befestigungsabschnitte geführt werden können.

Die Erfindung wird im Folgenden anhand von Ausführungsbeispielen unter Bezugnahme auf die Zeichnungen näher erläutert.
- Figur 1: zeigt eine perspektivische Ansicht einer Orthese, die nicht unter die Ansprüche fällt.
- Figur 2: zeigt eine weitere perspektivische Ansicht der Orthese.
- Figur 3: zeigt eine Ansicht von oben auf die Fingerkuppen einer Hand, an die die Orthese angelegt ist.
- Figur 4: zeigt eine Ansicht auf die Handinnenfläche der Hand mit der angelegten Orthese.
- Figur 5: zeigt eine Ansicht auf den Handrücken der Hand mit der angelegten Orthese.
- Figur 6: zeigt eine Ansicht auf die Daumen- und Zeigefingerseite der Hand mit der angelegten Orthese.
- Figur 7: zeigt eine perspektivische Ansicht einer ersten Ausführungsform der erfindungsgemäßen Orthese.
- Figur 8: zeigt eine weitere perspektivische Ansicht der ersten Ausführungsform der erfindungsgemäßen Orthese.
- Figur 9: zeigt eine perspektivische Ansicht von oben auf die Fingerkuppen einer Hand, an die die erste Ausführungsform der erfindungsgemäßen Orthese angelegt ist.
- Figur 10: zeigt eine perspektivische seitliche Ansicht der Hand mit der angelegten Orthese gemäß der ersten Ausführungsform mit vollständig geöffnetem Verschlusselement.
- Figur 11: zeigt eine Seitenansicht der Hand mit der angelegten Orthese gemäß der ersten Ausführungsform mit teilweise geschlossenem Verschlusselement.
- Figur 12: zeigt eine Seitenansicht der Hand mit der angelegten Orthese gemäß der ersten Ausführungsform mit vollständig geschlossenem Verschlusselement.
- Figur 13: zeigt eine Ansicht auf die Handinnenfläche der Hand mit der angelegten Orthese gemäß der ersten Ausführungsform.
- Figur 14: zeigt eine Ansicht auf die daumenabgewandte Handkante der Hand mit der angelegten Orthese gemäß der ersten Ausführungsform.
- Figur 15: zeigt eine perspektivische Ansicht einer zweiten Ausführungsform der erfindungsgemäßen Orthese.
- Figur 16: zweiten zeigt eine weitere perspektivische Ansicht der Ausführungsform der erfindungsgemäßen Orthese.
- Figur 17: zeigt eine weitere perspektivische Ansicht der zweiten Ausführungsform der erfindungsgemäßen Orthese.
- Figur 18: zeigt eine weitere perspektivische Ansicht der zweiten Ausführungsform der erfindungsgemäßen Orthese.
- Figur 19: zeigt eine Ansicht auf die Handinnenfläche einer Hand mit der angelegten Orthese gemäß der zweiten Ausführungsform.
- Figur 20: zeigt eine Ansicht auf den Handrücken der Hand mit der angelegten Orthese gemäß der zweiten Ausführungsform.
- Figur 21: zeigt eine Seitenansicht der Hand mit der angelegten Orthese gemäß der zweiten Ausführungsform.
- Figur 22: zeigt eine weitere Seitenansicht der Hand mit der angelegten Orthese gemäß der zweiten Ausführungsform.

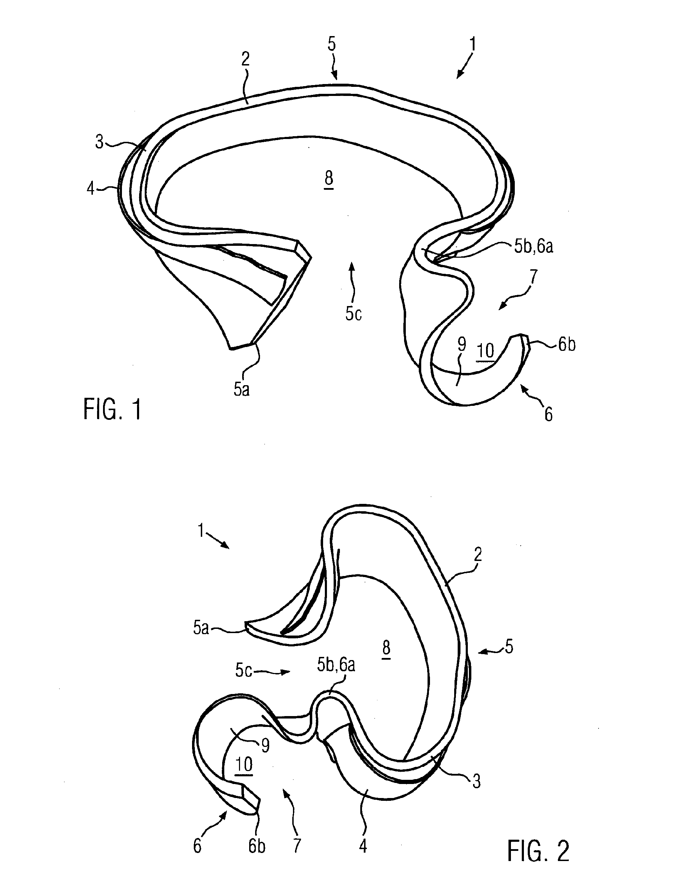

In den Figuren 1 und 2 sind perspektivische Ansichten einer Orthese 1 gezeigt, die zur Befestigung an einer menschlichen Hand angepasst ist, um im an die Hand angelegten Zustand Daumensattel- und -grundgelenk des Daumens der Hand ruhig zu stellen.
Die Orthese 1 besteht aus einem flachen, bandförmigen und gekrümmten steifen Körper 2 in Form eines länglichen Elements. Der steife Körper bzw. das längliche Element 2 weist ein steifes Kunststoffelement 3 und einen aus Aluminium ausgebildeten Verstärkungsstreifen 4 auf, der entlang eines Teils des Kunststoffelement 3 angebracht oder integriert ist.

Der steife Körper 2 und damit die Orthese 1 weisen zum einen einen Mittelhandabschnitt 5 auf, der in Form eines offenen und annähernd ovalen Rings zur Aufnahme der Mittelhand einer menschlichen Hand vorgesehen ist, und zum anderen einen Daumenabschnitt 6 mit einem ringssegmentförmigen Daumenhalteabschnitt 7 zur Aufnahme des Daumens der Hand. Der flache, bandförmige Körper 2 ist in der Weise zur Ausbildung der Orthese 1 gekrümmt, dass ein Teil einer ausgedehnten Seite des Körpers 2 die zur Anlage an einer Mittelhand vorgesehene Innenseite bzw. -fläche des Mittelhandabschnitts 5 bildet und ein Teil der gegenüberliegenden ausgedehnten Seite des Körpers 2 die zur Anlage an einem Daumen vorgesehene Innenseite bzw. -fläche des ringssegmentförmigen Daumenhalteabschnitts 7 bildet. Mit andern Worten sind die schmalen Längskanten des Körpers 2 in axialer Richtung des Mittelhandabschnitts 5 ausgerichtet.

Der Mittelhandabschnitt 5 hat ein erstes Ende 5a und ein zweites Ende 5b, die sich zur Ausbildung des offenen Rings durch einen Zwischenraum 5c getrennt gegenüberliegen. Das Ende 5a ist mit einem Längsende des Körpers 2 identisch, und der Mittelhandabschnitt 5 wird durch einen länglichen Teilabschnitt des länglichen Körpers 2 gebildet. Der durch den Mittelhandabschnitt 5 definierte längliche und im Wesentlichen ovale Innenraum 8 hat die typische Querschnittsform einer menschlichen Mittelhand. Die beiden Enden 5a und 5b und der Zwischenraum 5c sind auf einer der beiden ausgedehnten Seiten des Innenraums 8 angeordnet, so dass die gegenüberliegende ausgedehnte Seite des Innenraums 8 durchgehend von dem Körper 2 begrenzt wird.

Der Rest des Körpers 2 bildet den Daumenabschnitt 6, der damit ein weiterer länglicher Teilabschnitt des Körpers 2 ist, der sich unmittelbar an den den Mittelhandabschnitt 5 bildenden Teilabschnitt anschließt. Mit anderen Worten geht der Daumenabschnitt 6 von dem zweiten Ende 5b des Mittelhandabschnitts 5 aus und weist ein erstes Ende 6a und ein zweites Ende 6b auf, wobei das erste Ende 6a mit dem zweiten Ende 5b des Mittelhandabschnitts 5 zusammenfällt und das zweite Ende 6b des Daumenabschnitts 6 mit einem Längsende des Körpers 2 identisch ist. Der Daumenabschnitt 6 erstreckt sich im Wesentlichen senkrecht von dem Mittelhandabschnitt 5 und weist in der Nähe seines zweiten Endes 6b den ringssegmentförmigen Daumenhalteabschnitt 7 auf, der eine konkave Abstützfläche 9 zur Anlage an einem Daumen aufweist und einen Innenraum 10 zur Aufnahme des Daumens definiert. Der ringssegmentförmige Daumenhalteabschnitt 7 ist so angeordnet, dass der Innenraum 10 seitlich in einer Richtung weg von dem ersten Ende 5a des Mittelhandabschnitts 5 geöffnet ist.

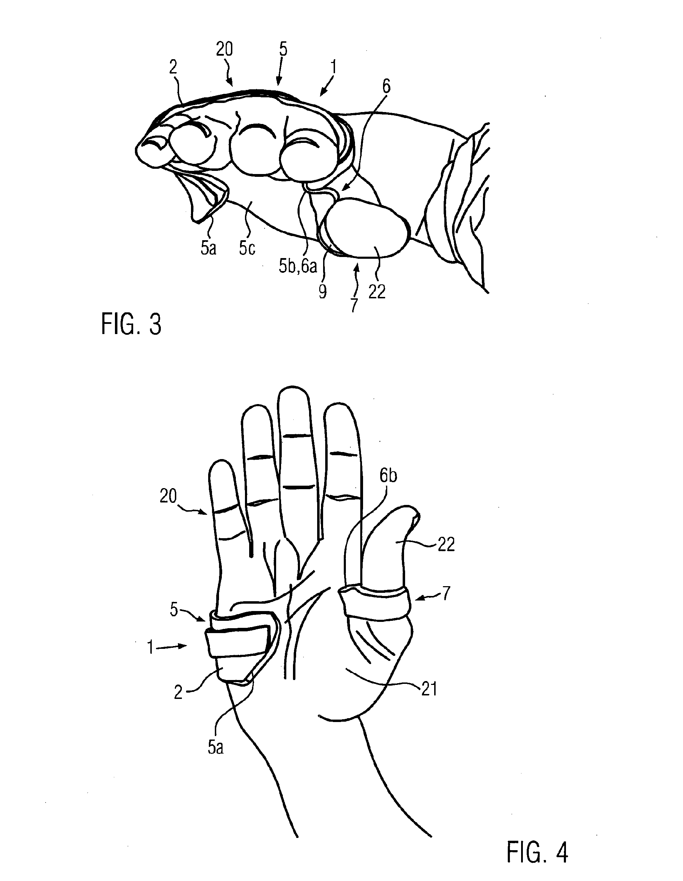

In Figur 3 ist der Verlauf des Körpers 2 der Orthese 1 gezeigt, nachdem die Orthese 1 an eine menschliche Hand 20 angelegt und genauer um die Mittelhand 21 und den Daumen 22 der Hand 20 herum gelegt worden ist (siehe Figuren 4 bis 6). Um sicherzustellen, dass der Körper 2 entlang des größten Teils seiner Länge an der Hand 20 anliegt, ist der Körper 2 mit einer gewissen Elastizität versehen.

Das erste Ende 5a ist im Bereich des kleinen Fingers und Ringfingers an der Innenfläche der Hand 20 angeordnet, und der den Mittelhandabschnitt 5 bildende längliche Abschnitt des Körpers 2 verläuft von dort um die äußere Handkante herum, über den gesamten Handrücken und um die innere Handkante herum, und das zweite Ende 5b des Mittelhandabschnitts 5 ist im Bereich des Daumens und Zeigefingers an der Innenfläche der Hand 20 angeordnet. Aufgrund der gewissen Elastizität des Mittelhandabschnitts 5 ist der Zwischenraum 5c bei dem Anlegen der Orthese 1 an die Hand 20 leicht aufgeweitet worden, und der Mittelhandabschnitt 5 liegt im Wesentlichen entlang seiner gesamten Länge an der Mittelhand 21 an.

Der Daumenabschnitt 6 erstreckt sich im Wesentlichen senkrecht von dem Mittelhandabschnitt 5 nach außen, so dass der ringssegmentförmige Daumenhalteabschnitt 7 so ausgerichtet ist, dass der Daumen 22 der Hand 20 in dem durch den Daumenhalteabschnitt 7 definierten Innenraum 10 angeordnet werden und die Innenseite des Daumens 22 an die Abstützfläche 9 angelegt werden kann. Es ist bevorzugt, wenn der ringssegmentförmige Daumenhalteabschnitt 7 so weit geschlossen ist, dass der Daumen 22 nach dem Anlegen der Orthese 1 nicht mehr ohne weiteres seitlich aus diesem heraus bewegt werden kann. Dazu ist es bevorzugt, wenn sich der ringssegmentförmige Daumenhalteabschnitt 7 über einen Winkel von mehr als 180° erstreckt und mehr als 50% des Umfangs des Daumens 22 umgreift. Zum Anlegen der Orthese 1 ist es dann erforderlich, vorzusehen, dass der Daumenhalteabschnitt 7 zum Anlegen elastisch ausreichend aufgeweitet werden kann oder dass der Daumen 22 zum Anlegen von unten in axialer Richtung in den Innenraum 10 eingeführt werden muss. Es sind dann keine separaten Verschlusselemente zur Sicherung der Orthese 1 an der Hand 20 erforderlich.

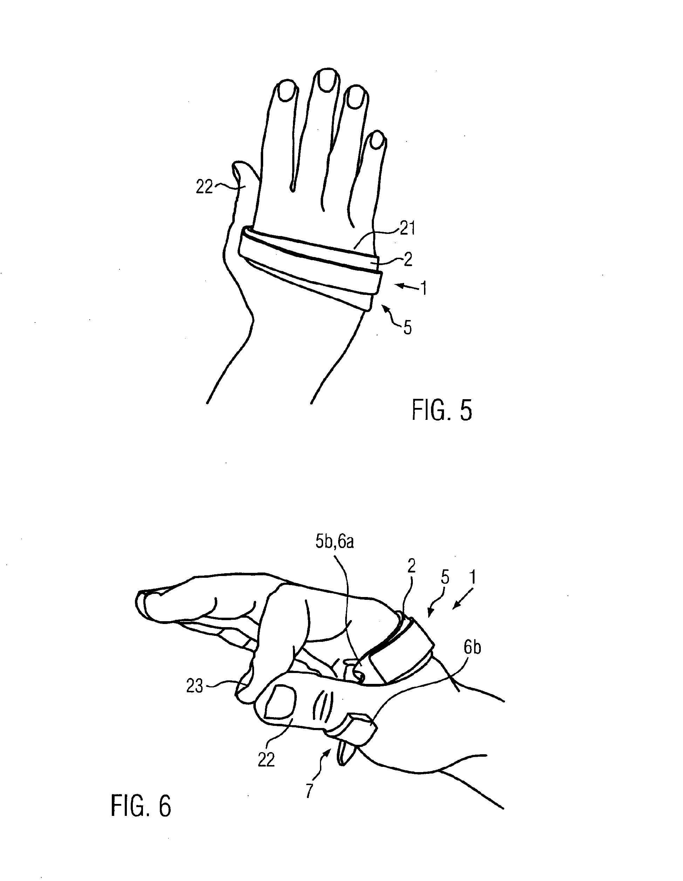

Die Figuren 4 und 5 zeigen Ansichten auf die Handfläche bzw. den Handrücken der Hand 20 mit angelegter Orthese 1. Es ist zu erkennen, dass sich der Mittelhandabschnitt 5 vollständig über den Handrücken erstreckt und beidseitig die Handkanten umgreift. Durch die Anordnung des Daumenhalteabschnitts 7 und die Ausrichtung der Abstützfläche 9 wird u.a. eine Abstützung des Daumens 22 in Bezug auf eine Bewegung in Richtung auf die Handinnenfläche bereitgestellt. Dennoch kann der wichtige Pinzettengriff ausgeführt werden, bei dem der Zeigefinger 23 mit dem Daumen 22 in Anlage gebracht wird (siehe Figur 6).

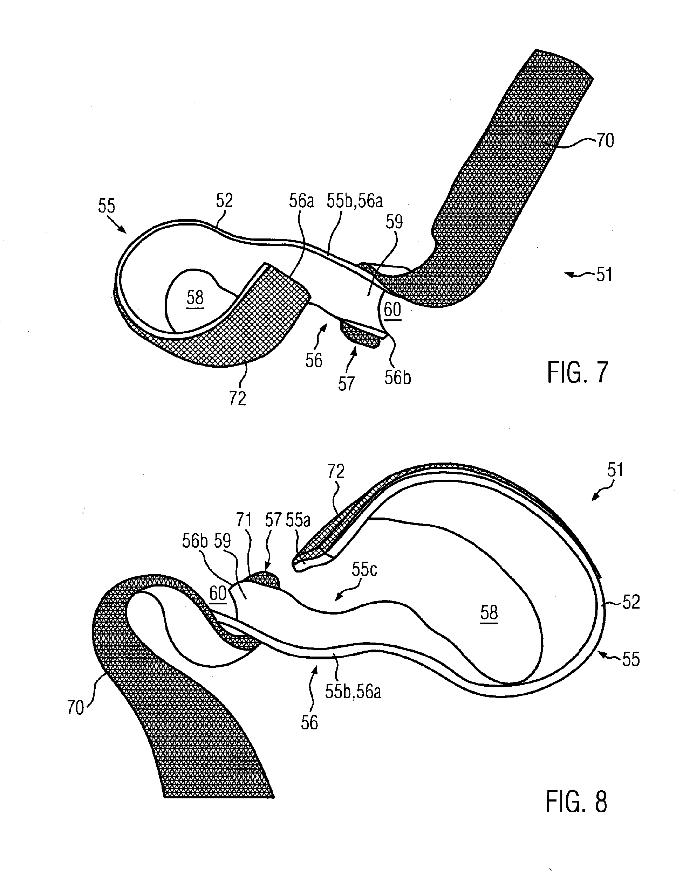

In den Figuren 7 und 8 sind perspektivische Ansichten einer ersten Ausführungsform einer Orthese 51 gezeigt, die zur Befestigung an einer menschlichen Hand angepasst ist, um im an die Hand angelegten Zustand Daumensattel- und -grundgelenk des Daumens der Hand ruhig zu stellen.

Die Orthese 51 weist wie die Orthese 1 einen flachen, bandförmigen und gekrümmten steifen Körper 52 in Form eines länglichen Elements auf. Der steife Körper bzw. das längliche Element 52 ist aus einem Kunststoffmaterial ausgebildet. Zur Verstärkung kann, zum Beispiel in seinem Inneren, ein Verstärkungselement aus Aluminium vorgesehen sein.

Der Grundaufbau der Orthese 51 und des Körpers 52 entspricht der Ausgestaltung der Orthese 1 und des Körpers 2.

Dementsprechend weisen der steife Körper 52 und damit die Orthese 51 zum einen einen Mittelhandabschnitt 55 auf, der in Form eines offenen und annähernd ovalen Rings zur Aufnahme der Mittelhand einer menschlichen Hand vorgesehen ist, und zum anderen einen Daumenabschnitt 56 mit einem ringssegmentförmigen Daumenhalteabschnitt 57 zur Aufnahme des Daumens der Hand. Der flache, bandförmige Körper 52 ist in der Weise zur Ausbildung der Orthese 51 gekrümmt, dass ein Teil einer ausgedehnten Seite des Körpers 52 die zur Anlage an einer Mittelhand vorgesehene Innenseite bzw. -fläche des Mittelhandabschnitts 55 bildet und ein Teil der gegenüberliegenden ausgedehnten Seite des Körpers 52 die zur Anlage an einem Daumen vorgesehene Innenseite bzw. -fläche des ringssegmentförmigen Daumenhalteabschnitts 57 bildet. Mit andern Worten sind die schmalen Längskanten des Körpers 52 in axialer Richtung des Mittelhandabschnitts 55 ausgerichtet.

Dabei hat der Mittelhandabschnitt 55 ein erstes Ende 55a und ein zweites Ende 55b, die sich zur Ausbildung des offenen Rings durch einen Zwischenraum 55c getrennt gegenüberliegen. Das Ende 55a ist mit einem Längsende des Körpers 52 identisch, und der Mittelhandabschnitt 55 wird durch einen länglichen Teilabschnitt des länglichen Körpers 52 gebildet. Der durch den Mittelhandabschnitt 55 definierte längliche und im Wesentlichen ovale Innenraum 58 hat die typische Querschnittsform einer menschlichen Mittelhand.

Im Unterschied zum Fall der Orthese 1 sind die beiden Enden 55a und 55b und der Zwischenraum 55c jedoch auf einer der beiden kurzen Seiten des Innenraums 58 angeordnet.

Der Rest des Körpers 52 bildet wiederum den Daumenabschnitt 56, der damit ein weiterer länglicher Teilabschnitt des Körpers 52 ist, der sich unmittelbar an den den Mittelhandabschnitt 55 bildenden Teilabschnitt anschließt. Mit anderen Worten geht der Daumenabschnitt 56 von dem zweiten Ende 55b des Mittelhandabschnitts 55 aus und weist ein erstes Ende 56a und ein zweites Ende 56b auf, wobei das erste Ende 56a mit dem zweiten Ende 55b des Mittelhandabschnitts 55 zusammenfällt und das zweite Ende 56b des Daumenabschnitts 56 mit einem Längsende des Körpers 52 identisch ist. Der Daumenabschnitt 56 erstreckt sich anders als bei der Orthese 1 im Wesentlichen in derselben Richtung wie der Mittelhandabschnitt 55 an dessen zweitem Ende 55b. Er weist in der Nähe seines zweiten Endes 56b den ringsegmentförmigen Daumenhalteabschnitt 57 auf, der eine konkave Abstützfläche 59 zur Anlage an einem Daumen aufweist und einen Innenraum 60 zur Aufnahme des Daumens definiert.

Der Daumenabschnitt 56 ist insgesamt in der Art einer Schiene ausgebildet, an die ein Daumen entlang des größten Teils seiner Länge beginnend mit dem Daumengrundgelenk angelegt werden kann. Die Abstützfläche 59 des Daumenhalteabschnitts 57 erstreckt sich von diesem bis über den Rest des Daumenabschnitts 56. Der ringssegmentförmige Daumenhalteabschnitt 57 - und der Daumenabschnitt 56 insgesamt - ist dabei so angeordnet, dass der Innenraum 60 seitlich in einer Richtung hin zu dem ersten Ende 55a des Mittelhandabschnitts 55 geöffnet ist.

An dem Daumenhalteabschnitt 57 ist ein Klettband 70 befestigt, und zwar in der Weise, dass sein eines Ende mit einem längs Ende des ringssegmentförmigen Daumenhalteabschnitts 57 ausgerichtet ist und das Klettband 70 entlang der gesamten Außenfläche des Daumenhalteabschnitts 57, die der Abstützfläche 59 gegenüberliegt, an diesem befestigt ist. Zu diesem Zweck ist an dieser Außenfläche ein Klettelement 71 angebracht. Ein weiteres Klettelement 72 ist entlang eines Teils der Außenfläche des Mittelhandabschnitts 55 angebracht, und zwar in der Nähe des Endes 55a.

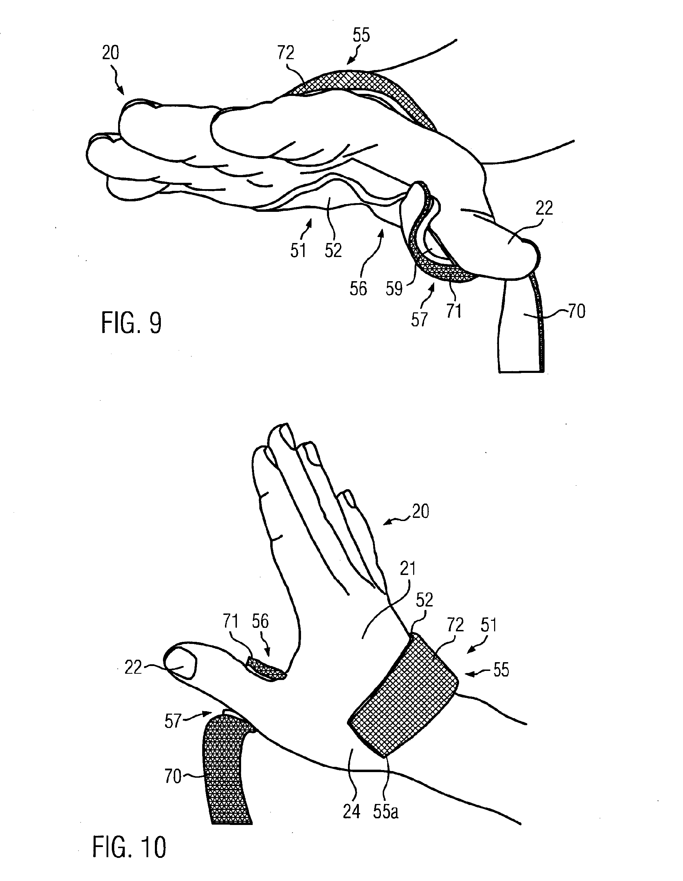

Der Verlauf des Körpers 52 der Orthese 51 nach dem Anlegen an eine menschliche Hand 20 und genauer um die Mittelhand 21 und den Daumen 22 der Hand 20 herum ist aus den Figuren 9 und 10 ersichtlich. Um sicherzustellen, dass der Körper 52 entlang des größten Teils seiner Länge an der Hand 20 anliegen kann, kann er ebenfalls mit einer gewissen Elastizität versehen sein.

Das erste Ende 55a ist im Bereich des Daumensattelgelenks 24 am Handrücken der Hand 20 angeordnet, und der den Mittelhandabschnitt 55 bildende längliche Abschnitt des Körpers 52 verläuft von dort über den Handrücken in Richtung auf die äußere Handkante, um diese Handkante herum und über die Handinnenfläche in Richtung auf die innere Handkante, und das zweite Ende 55b des Mittelhandabschnitts 55 ist im Bereich des Daumensattelgelenks 24 an der Innenfläche der Hand 20 angeordnet. Der Mittelhandabschnitt 55 ist anatomisch entsprechend der typischen Form einer menschlichen Mittelhand vorgeformt, und aufgrund einer gewissen Elastizität des Mittelhandabschnitts 55 ist der Zwischenraum 55c bei dem Anlegen der Orthese 51 an die Hand 20 leicht aufgeweitet worden. Nach der endgültigen Befestigung der Orthese 51 mit Hilfe des Klettbandes 70 liegt der Mittelhandabschnitt 55 im Wesentlichen entlang seiner gesamten Länge an der Mittelhand 21 an.

Der Daumenabschnitt 56 erstreckt sich im Wesentlichen in der Verlängerung der Handinnenfläche von dem Mittelhandabschnitt 55 nach außen, so dass der Daumenabschnitt 56 und insbesondere der ringsegmentförmige Daumenhalteabschnitt 57 so ausgerichtet ist, dass der Daumen 22 der Hand 20 an den Daumenabschnitt 56 angelegt und in dem durch den Daumenhalteabschnitt 57 definierten Innenraum 60 angeordnet werden kann und dass dabei die Innenseite des Daumens 22 an die Abstützfläche 59 angelegt werden kann. Für die Ausgestaltung des ringssegmentförmigen Daumenhalteabschnitts 57 gelten ansonsten dieselben Erwägungen wie für den Daumenhalteabschnitt 7 der Orthese 1.

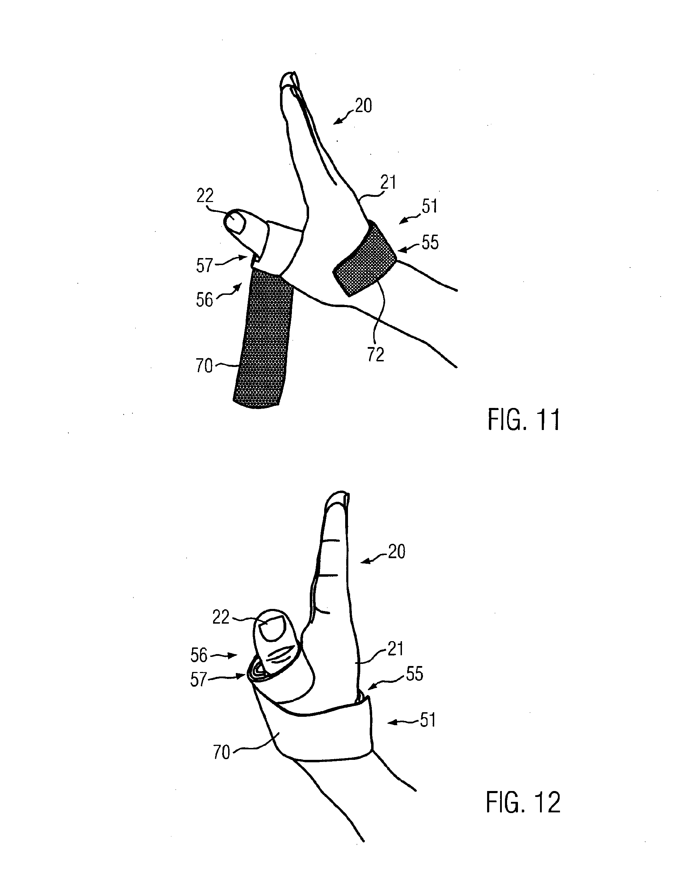

Aufgrund des Vorsehens des Klettbandes 70 ist es aber nicht erforderlich, aber möglich, dass sich der ringsegmentförmige Daumenhalteabschnitt 57 über einen Winkel von mehr als 180° erstreckt. Zum Sichern der Orthese 51 an der Hand 20 wird das Klettband 70 in einem ersten Schritt über die seitliche Öffnung in dem ringsegmentförmigen Daumenhalteabschnitt 57 gelegt (siehe Figur 11). Anschließend wird das Klettband 70 in Richtung auf das erste Ende 55a des Mittelhandabschnitts 55 geführt und unter Spannung an dem Klettelement 72 befestigt (siehe Figur 12).

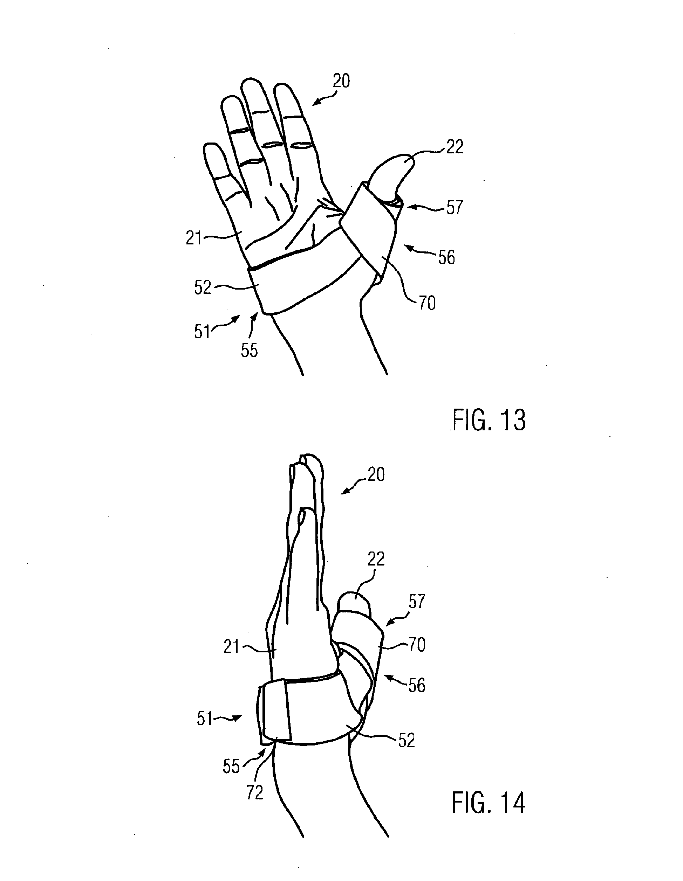

Die Figuren 13 und 14 zeigen Ansichten auf die Handfläche bzw. die äußere Handkante der Hand 20 mit angelegter und durch das Klettband 70 befestigter Orthese 51. Es ist zu erkennen, dass sich der Mittelhandabschnitt 55 vollständig über die Handinnenfläche erstreckt und die äußere Handkante umgreift. Durch die Anordnung des Daumenabschnitts 56 und insbesondere des Daumenhalteabschnitts 57 und die Ausrichtung der Abstützfläche 59 wird wieder u.a. eine Abstützung des Daumens 22 in Bezug auf eine Bewegung in Richtung auf die Handinnenfläche bereitgestellt. Dennoch kann der wichtige Pinzettengriff ausgeführt werden.

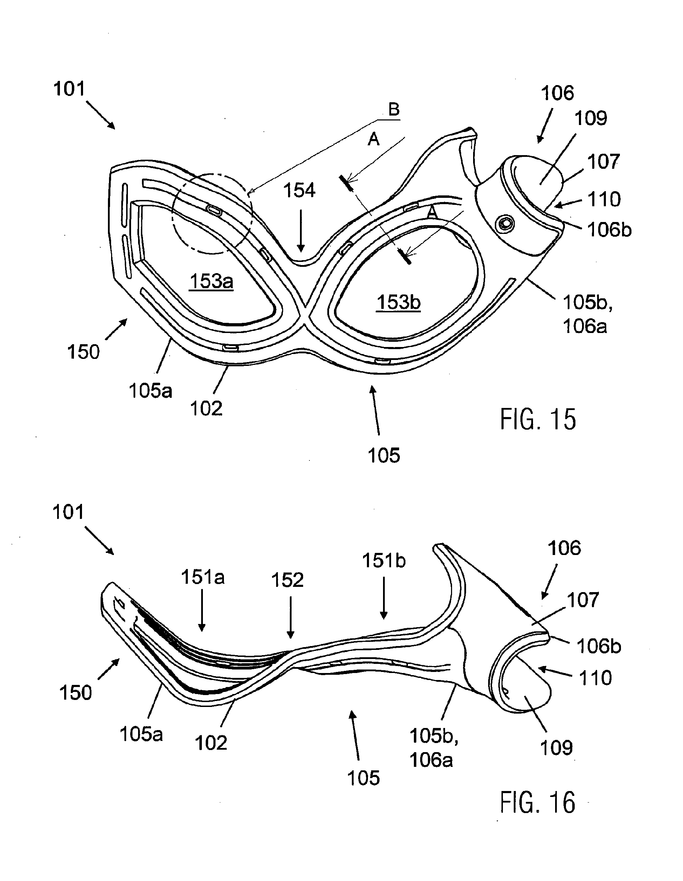

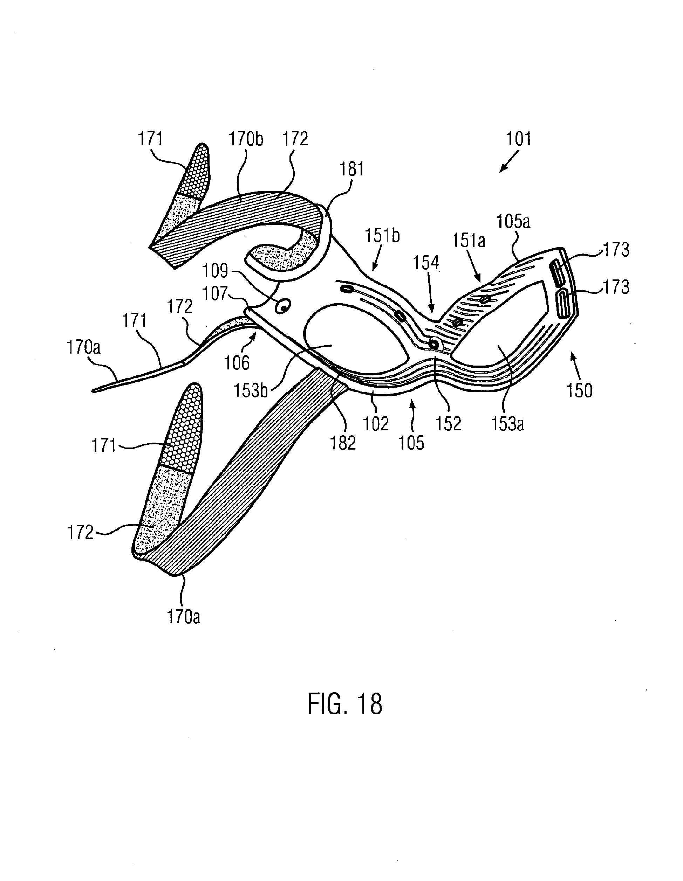

In den Figuren 15 bis 18 sind perspektivische Ansichten einer zweiten Ausführungsform einer Orthese 101 gezeigt, die zur Befestigung an einer menschlichen Hand angepasst ist, um im an die Hand angelegten Zustand Daumensattel- und -grundgelenk des Daumens der Hand ruhig zu stellen. Dabei ist die Orthese 101 in den Figuren 15 bis 17 in einer Variante ohne Verschlusselemente und in der Figur 18 in einer anderen Variante mit Befestigungsbändern gezeigt. In der ersten Variante sind ebenfalls Befestigungsbänder vorgesehen, die zum Beispiel in derselben Weise ausgestaltet sein können, wie die in Figur 18 gezeigten Befestigungsbänder, jedoch in den Figuren 15 bis 17 nicht gezeigt sind und bei der Benutzung der Orthese 101 mit dem Rest der Orthese 101 verbunden werden müssen. Mit Ausnahme der Befestigungsbänder weist die Orthese 101 in beiden Varianten genau dieselbe Konstruktion und Ausgestaltung auf, so dass nachfolgend nur noch in Bezug auf die Befestigungsbänder zwischen ihnen unterschieden wird.

Die Orthese 101 weist einen flachen, bandförmigen und gekrümmten steifen Körper 102 in Form eines länglichen Elements auf. Der steife Körper bzw. das längliche Element 102 ist aus einem Kunststoffmaterial ausgebildet. Zur Verstärkung kann, zum Beispiel in seinem Inneren, ein Verstärkungselement aus Aluminium vorgesehen sein.

Der Grundaufbau der Orthese 101 und des Körpers 102 entspricht der Ausgestaltung der Orthesen 1 und 51 und der Körper 2 und 52. Dementsprechend weisen der steife Körper 102 und damit die Orthese 101 zum einen einen Mittelhandabschnitt 105 auf und zum anderen einen Daumenabschnitt 106 mit einem ringssegmentförmigen Daumenhalteabschnitt 107 zur Aufnahme des Daumens der Hand.

Im Unterschied zu den vorhergehenden Ausführungsformen ist der Mittelhandabschnitt 105 aber so dimensioniert, dass er nach bestimmungsgemäßer Anlage an die Handinnenfläche einer Hand nicht über den Handrücken verläuft. Dabei ist der flache, bandförmige Körper 102 in der Weise zur Ausbildung der Orthese 101 gekrümmt, dass ein Teil einer ausgedehnten Seite des Körpers 102 sowohl die zur Anlage an der Handinnenfläche einer Mittelhand vorgesehene Innenseite bzw. -fläche des Mittelhandabschnitts 105 bildet (die im Folgenden als palmare Fläche bezeichnet wird), als auch die zur Anlage an einem Daumen vorgesehene Innenseite bzw. -fläche des ringssegmentförmigen Daumenhalteabschnitts 107 bildet. Die schmalen Längskanten des Körpers 102 sind in axialer Richtung des Mittelhandabschnitts 105 ausgerichtet.

Der Mittelhandabschnitt 105 hat ein erstes Ende 105a und ein von diesem beabstandetes zweites Ende 105b. Das Ende 105a ist mit einem Längsende des Körpers 102 identisch, und der Mittelhandabschnitt 105 wird durch einen länglichen Teilabschnitt des länglichen Körpers 102 gebildet.

An das erste Ende 105a des Körpers 102 schließt sich einstückig ein flexibler Abschnitt 150 an. Dazu werden der Körper 102 und der flexible Abschnitt 150 beispielsweise durch ein einziges flexibles Kunststoffelement gebildet, wobei zur Ausbildung des Körpers 102 in dessen Bereich in dem Kunststoffelement eine Verstärkung oder Versteifung vorgesehen ist.

Der Rest des Körpers 102 bildet wiederum den Daumenabschnitt 106, der damit ein weiterer - ggf. ebenfalls länglicher - Teilabschnitt des Körpers 102 ist, der sich unmittelbar an den den Mittelhandabschnitt 105 bildenden Teilabschnitt anschließt. Mit anderen Worten geht der Daumenabschnitt 106 von dem zweiten Ende 105b des Mittelhandabschnitts 105 aus und weist ein erstes Ende 106a und ein zweites Ende 106b auf, wobei das erste Ende 106a mit dem zweiten Ende 105b des Mittelhandabschnitts 105 zusammenfällt und das zweite Ende 106b des Daumenabschnitts 106 mit einem Längsende des Körpers 102 identisch ist. Der Daumenabschnitt 106 erstreckt sich wie bei der Orthese 51 im Wesentlichen in derselben Richtung wie der Mittelhandabschnitt 105 an dessen zweitem Ende 105b. Er weist in der Nähe seines zweiten Endes 106b den ringsegmentförmigen Daumenhalteabschnitt 107 auf, der eine konkave Abstützfläche 109 zur Anlage an einem Daumen aufweist und einen Innenraum 110 zur Aufnahme des Daumens definiert.

Der Daumenabschnitt 106 stellt eine Schiene bereit, an die ein Daumen entlang eines Teils seiner Länge beginnend mit dem Daumengrundgelenk angelegt werden kann. Die Abstützfläche 109 des Daumenhalteabschnitts 107 erstreckt sich von diesem bis über den Rest des Daumenabschnitts 106. Der ringssegmentförmige Daumenhalteabschnitt 107 - und der Daumenabschnitt 106 insgesamt - ist dabei so angeordnet, dass der Innenraum 110 seitlich in einer Richtung hin zu dem ersten Ende 105a des Mittelhandabschnitts 105 geöffnet ist, und zwar auf der Seite des Körpers 102, auf der sich auch die palmare Fläche befindet.

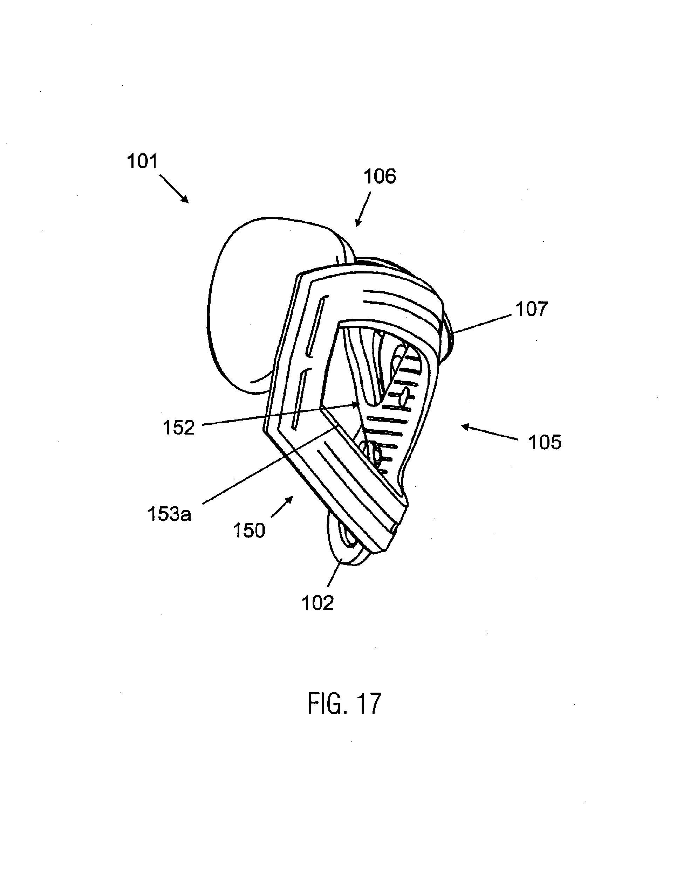

Der Mittelhandabschnitt 105 ist so gekrümmt, dass in der palmaren Fläche zwei konkave Abschnitte 151a, 151b und in Richtung von dem ersten Ende 105a zum zweiten Ende 105b zwischen ihnen ein konvexer Abschnitt 152 (siehe auch Figur 17) vorgesehen ist. Der konvexe Abschnitt 152 stellt nach Anlage an eine Hand eine Auswölbung bereit, die der Mulde in der Handinnenfläche zwischen dem Ende des auslaufenden Daumenballens und dem Abschnitt in der Nähe der gegenüberliegenden Handkante entspricht, und die konkaven Abschnitte 151a, 151b entsprechend der Handstruktur angrenzend an die Mulde. Dadurch kann der Mittelhandabschnitt 105 eng und sicher an der Handinnenfläche anliegen.

Um den Tragekomfort zu verbessern und das Gewicht der Orthese 101 zu verringern, sind im Bereich der konkaven Abschnitt 151a, 152b jeweilige Durchgangsöffnungen 153a, 153b durch den Mittelhandabschnitt 105 ausgebildet, wobei die Durchgangsöffnung 153a zum Teil auch durch den flexiblen Abschnitt 150 verläuft und durch diesen begrenzt wird. Außerdem weist der Mittelhandabschnitt 105 im Bereich des konvexen Abschnitts 152 an den beiden Längskanten eine Einschnürung bzw. Verjüngung 154 auf, durch die der Mittelhandabschnitt 105 zusammen mit dem flexiblen Abschnitt 150 die Form einer "8" oder eines "B" hat bzw. brillenförmig ist, wobei die Durchgangsöffnungen 153a, 153b bevorzugt durch im Vergleich zu ihrem Durchmesser schmalere Ringe begrenzt werden, die durch den Mittelhandabschnitt 105 und ggf. angrenzende Abschnitte der Orthese gebildet werden, wie den Daumenabschnitt 106 und/oder den flexiblen Abschnitt 150. Durch das Vorsehen der Verjüngung 154 kann einem Abheben des Mittelhandabschnitts 105 bei Handbewegungen entgegengewirkt werden.

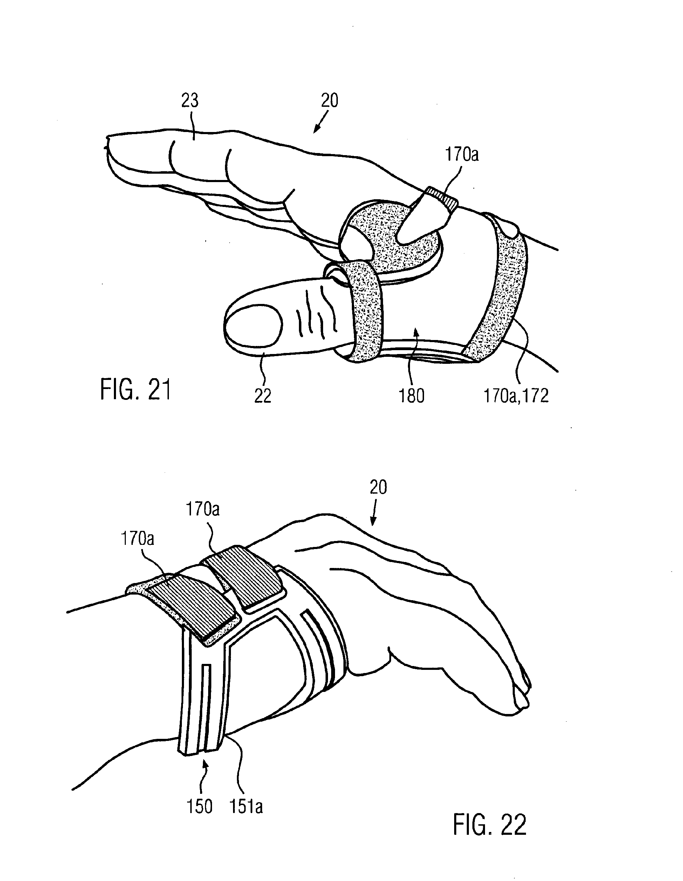

Wie in Figur 18 gezeigt ist, weist die Orthese 101 als Verschlusseinrichtung zur Befestigung der Orthese 101 im angelegten Zustand an einer Hand zwei erste Klettbänder 170a und ein zweites Klettband 170b auf. Jedes Klettband 170a, 170b weist in einem Endbereich ein Klettelement 171 und zumindest entlang eines Teils seiner restlichen Länge ein weiteres Klettelement 172 auf, wobei die Klettelemente 171 und 172 lösbar aneinander befestigt werden können. Die Klettbänder 170a, 170b sind an einem Ende jeweils dauerhaft an dem Mittelhandabschnitt 105 oder dem Daumenabschnitt 106 befestigt. In der Variante der Figuren 15 bis 17 sind die dort nicht dargestellten Klettbänder 170a, 170b dagegen von dem Mittelhandabschnitt 105 bzw. dem Daumenabschnitt 106 insgesamt lösbar und können, zum Beispiel in der in der Figur 18 bzw. den Figuren 19 bis 22 gezeigten Position, an dem Mittelhandabschnitt 105 bzw. dem Daumenabschnitt 106 zur Fixierung der Orthese 101 an einer Hand befestigt werden.

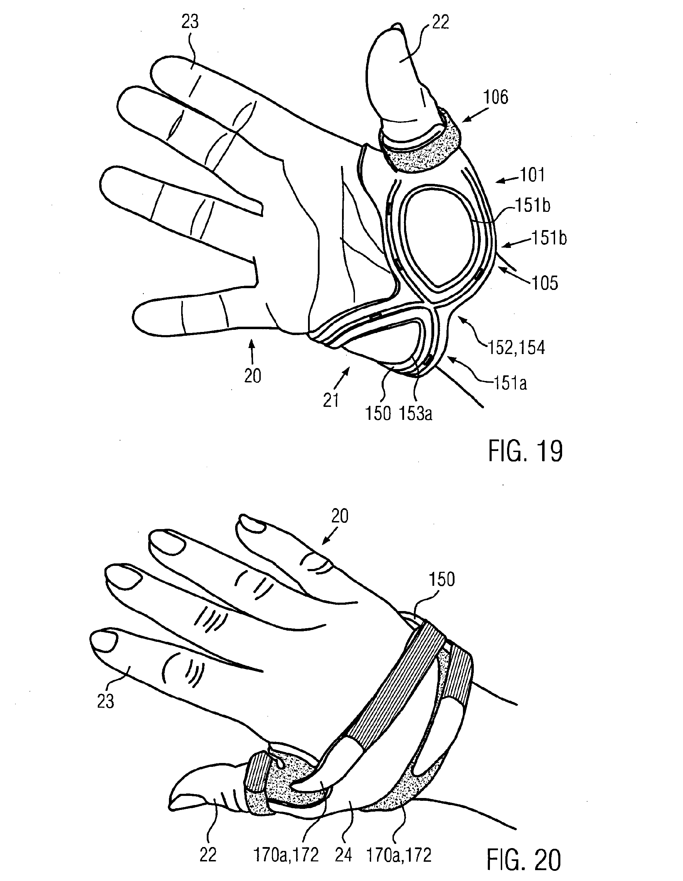

Die Figuren 19 bis 22 zeigen die Orthese 101 im an eine menschliche Hand 20 angelegten Zustand. Um sicherzustellen, dass der Körper 102 entlang des größten Teils seiner Länge an der Hand 20 anliegen kann, kann er wiederum mit einer gewissen Elastizität versehen sein.

Der Mittelhandabschnitt 105 erstreckt sich im Bereich der Mittelhand 21 quer über die Handinnenfläche der Hand 20, wobei sein erstes Ende 105a in der Nähe der äußeren Handkante, aber von dieser beabstandet angeordnet ist, und sein zweites Ende 105b im Bereich des Daumensattelgelenks 24 angeordnet ist. Dabei ist ein Teil des auslaufenden Daumenballens im Bereich des konkaven Abschnitts 151b und der Durchgangsöffnung 153b angeordnet, und der konkave Abschnitt 151a und die Durchgangsöffnung 153a nehmen einen Bereich in der Nähe der äußeren Handkante auf. Die dazwischen befindliche Vertiefung in der Handinnenfläche nimmt die durch den konvexen Abschnitt 152 bereitgestellte Auswölbung auf. Der Mittelhandabschnitt 105 ist somit anatomisch entsprechend der typischen Form einer menschlichen Mittelhand vorgeformt, kann aber bevorzugt eine gewisse Elastizität und/oder eine gewisse plastische Verformbarkeit aufweisen, um eine Anpassung an verschiedene Hände 20 zu verbessern.

Der an das erste Ende 105a angrenzende flexible Abschnitt 150 verläuft um die äußere Handkante herum und in Anlage an dieser, und auf der anderen Seite erstreckt sind der Daumenabschnitt 106 im Wesentlichen in der Verlängerung der Handinnenfläche von dem Mittelhandabschnitt 105 nach außen, so dass der Daumenabschnitt 106 und insbesondere der ringsegmentförmige Daumenhalteabschnitt 107 so ausgerichtet ist, dass der Daumen 22 der Hand 20 an den Daumenabschnitt 106 angelegt und in dem durch den Daumenhalteabschnitt 107 definierten Innenraum 110 angeordnet werden kann und dass dabei die Innenseite des Daumens 22 an die Abstützfläche 109 angelegt werden kann. Für die Ausgestaltung des ringssegmentförmigen Daumenhalteabschnitts 107 gelten ansonsten dieselben Erwägungen wie für die Daumenhalteabschnitte 7 bzw. 57 der Orthesen 1 und 51.

Zur Fixierung der Orthese 101 an der Hand 20 wird jedes der Klettbänder 170a ausgehend von dessen Befestigungsstelle an dem Daumenabschnitt 106 bzw. dem Mittelhandabschnitt 105 in der Nähe des Daumenabschnitts 106 nach dem Anlagen der Orthese 101 an die Hand 20 über den Handrücken der Hand 20 gelegt und mit dem das Klettelement 171 aufweisenden Ende durch eine jeweilige zugeordnete Öffnung 173 geführt, die in dem vom Mittelhandabschnitt 105 abgewandten Endbereich des flexiblen Abschnitts 150 vorgesehen ist. Anschließend wird jedes Klettband 170a zur Ausbildung einer Schlaufe wieder zurückgeführt, sein Klettelement 171 wird so an seinem Klettelement 172 befestigt, dass das Klettband 170a gespannt ist. In diesem Zustand bilden die Klettbänder 170a, der Mittelhandabschnitt 105, der Daumenabschnitt 106 und der flexible Abschnitt 150 eine ringförmig geschlossene Struktur, die die Hand 20 umschließt und die Orthese 101 and der Hand 20 festhält. Die Befestigungsstelle des einen der beiden Klettbänder 170a kann zum Beispiel an einem Bereich 181 des Daumenabschnitts 106 bzw. des Mittelhandabschnitts 105 zur Anlage zwischen Daumen 22 und Zeigefinger 23 angeordnet sein, und die Befestigungsstelle des anderen der beiden Klettbänder 170a kann zum Beispiel in einem Bereich 182 des Mittelhandabschnitts 105 zur Anlage im Bereich des Daumensattelgelenks 24 angeordnet sein.

Außerdem wird zur Fixierung der Orthese 101 an der Hand 20 das Klettband 107b ausgehend von dessen Befestigungsstelle an dem Daumenhalteabschnitt 107 über den durch dessen ringsegmentförmige Ausbildung bedingten Zwischenraum 180 gelegt, und dann wird das Klettelement 171 an dem Klettelement 172 befestigt, so dass das Klettband 107b zusammen mit dem Daumenhalteabschnitt 107 den Daumen 22 ringförmig vollständig umschließt.

Durch die Anordnung des Daumenabschnitts 106 und insbesondere des Daumenhalteabschnitts 107 und die Ausrichtung der Abstützfläche 109 wird wieder u.a. eine Abstützung des Daumens 22 in Bezug auf eine Bewegung in Richtung auf die Handinnenfläche bereitgestellt. Dennoch kann der wichtige Pinzettengriff ausgeführt werden.

## Patentansprüche

1. Orthese zur Befestigung an einer menschlichen Hand (20) zur Immobilisierung von Daumensattel- und -grundgelenk des Daumens (22) der Hand (20) mit einem steifen Körper (2, 52, 102), mit
einem Mittelhandabschnitt (5, 55, 105), der durch einen Abschnitt eines gekrümmten und steifen Elements gebildet wird und der
- ein erstes Ende (5a, 55a, 105a) und ein zweites Ende (5b, 55b, 105b) aufweist, die voneinander beabstandet sind, und
- in der Weise ausgebildet ist, dass er mit einer menschlichen Hand (20) in Eingriff gebracht werden kann, indem er unterhalb der Fingergrundgelenke von Zeigefinger, Mittelfinger, Ringfinger und kleinem Finger so an die Handinnenfläche der Hand (20) angelegt wird, dass er sich in einer Richtung zwischen den beiden Handkanten über zumindest einen Teil der Handinnenfläche erstreckt und zumindest teilweise an dieser anliegt, und
einem steifen Daumenabschnitt (6, 56, 106), der
- sich von dem Mittelhandabschnitt (5, 55, 105) erstreckt und ein erstes Ende (6a, 56a, 106a), das an dem Mittelhandabschnitt (5, 55, 105) befestigt ist oder durch einen Teil des Mittelhandabschnitts (5, 55, 105) gebildet wird, und ein zweites Ende (6b, 56b, 106b) aufweist,
- im Bereich seines zweiten Endes (6b, 56b, 106b) einen ringförmigen oder ringsegmentförmigen Daumenhalteabschnitt (7, 57, 107) aufweist und
- in der Weise ausgebildet ist, dass der Daumenabschnitt (6, 56, 106) bei mit der Hand (20) in Eingriff gebrachtem Mittelhandabschnitt (5, 55, 105) so an dem Daumen (22) der Hand (20) angelegt werden kann, dass
- der Daumen (22) in der durch den Daumenhalteabschnitt (7, 57, 107) definierten Ringöffnung (10, 60, 110) angeordnet ist und der Daumenhalteabschnitt (7, 57, 107) sich über zumindest einen Teil des Umfangs des Daumens (22) erstreckt und ihn zumindest teilweise umfasst und
- den Daumen (22) gegen eine Bewegung in Richtung auf die Handfläche in einer Position abstützt, in der der Daumen (22) und der Zeigefinger (23) der Hand (20) gegenübergestellt sind und der Zeigefinger (23) in Richtung auf den Daumen (22) in Anlage an diesen und von dem Daumen (22) weg bewegt werden kann,
wobei der Mittelhandabschnitt (105) in der Weise ausgebildet ist, dass er im an eine menschliche Hand (20) angelegten Zustand der Orthese (101), in dem der Mittelhandabschnitt (105) mit der Hand (20) in Eingriff gebracht worden ist und der Daumenabschnitt (106) so an dem Daumen (22) der Hand (20) angelegt worden ist, dass der Daumen (22) in der durch den Daumenhalteabschnitt (107) definierten Ringöffnung (110) angeordnet ist und der Daumenhalteabschnitt (107) sich über zumindest einen Teil des Umfangs des Daumens (22) erstreckt und ihn zumindest teilweise umfasst, ausschließlich im Bereich der Handinnenfläche an der Hand (20) anliegt, und
wobei die Orthese ferner eine Verschlusseinrichtung (170a, 170b) zum Befestigen der Orthese (101) in dem angelegten Zustand an der Hand (20) aufweist, wobei die Verschlusseinrichtung (170a, 170b) mindestens einen ersten Befestigungsabschnitt (170a) aufweist, der so angeordnet und ausgestaltet ist, dass er in dem angelegten Zustand der Orthese (101) derart angeordnet und an dem Mittelhandabschnitt (105) und/oder dem Daumenabschnitt (106) fixiert werden kann, dass er über den Handrücken der Hand (20) verläuft und zusammen mit dem Mittelhandabschnitt (105) zumindest einen Teil eines ringförmig geschlossenen Abschnitts der Orthese (51) bildet, der die Hand (20) umschließt, und
wobei der Mittelhandabschnitt (105) eine erste Fläche zur Anlage an der Handinnenfläche und eine gegenüberliegende zweite Fläche aufweist, die in dem angelegten Zustand der Orthese von der Hand (20) weg gerichtet ist, wobei die erste Fläche zwischen dem ersten (105a) und zweiten Ende (105b) des Mittelhandabschnitts (105) zwei konkave Abschnitte (151a, 151b) und einen dazwischen angeordneten konvexen Abschnitt (152) aufweist.

2. Orthese nach Anspruch 1, bei der
- das gekrümmte und steife Element und dessen Abschnitt, der den Mittelhandabschnitt (5, 55) bildet, länglich sind,
- das erste Ende (5a, 55a) des Mittelhandabschnitts (5, 55) durch eines der beiden Längsenden des länglichen Elements gebildet wird und der Mittelhandabschnitt (5, 55) das erste Ende (5a, 55a) und das zweite Ende (5b, 55b) in Längsrichtung aufweist,
- der Mittelhandabschnitt (5, 55) die Form eines Segments eines eine längliche Ringöffnung (8, 58) umschließenden Rings hat, so dass das erste und das zweite Ende (5a, 55a; 5b, 55b) des Mittelhandabschnitts (5, 55) voneinander beabstandet sind,
- der Mittelhandabschnitt (5, 55) in der Weise ausgebildet ist, dass er mit einer menschlichen Hand (20) in Eingriff gebracht werden kann, indem er unterhalb der Fingergrundgelenke von Zeigefinger, Mittelfinger, Ringfinger und kleinem Finger so um den Handrücken und die Handinnenfläche der Hand (20) gelegt wird, dass er
- sich in einer Richtung zwischen den beiden Handkanten über zumindest einen Teil des Handrückens erstreckt und zumindest teilweise an diesem anliegt,
- um mindestens eine der beiden Handkanten verläuft und zumindest teilweise an diesen anliegt und
- sich in einer Richtung zwischen den beiden Handkanten über zumindest einen Teil der Handinnenfläche erstreckt und zumindest teilweise an dieser anliegt, und
der Daumenabschnitt (6, 56) länglich ist und das erste Ende (6a, 56a) und zweite Ende (6b, 56b) des Daumenabschnitts (6, 56) in dessen Längsrichtung aufweist.

3. Orthese nach Anspruch 1 oder Anspruch 2, bei der der steife Körper (2, 52) aus dem Element ausgebildet ist und der Daumenabschnitt (6, 56) durch einen Abschnitt des Elements gebildet wird, wobei sich der Daumenabschnitt (6, 56) von dem zweiten Ende (5b, 55b) des Mittelhandabschnitts (5, 55) erstreckt und das erste Ende (6a, 56a) des Daumenabschnitts (6, 56) an dem zweiten Ende (5b, 55b) des Mittelhandabschnitts (5, 55) befestigt ist oder durch dieses gebildet wird.

4. Orthese nach einem der vorhergehenden Ansprüche, bei der in dem Bereich des zweiten Endes (5b, 55b) des Mittelhandabschnitts (5, 55) und/oder des erstes Endes (6a, 56a) des Daumenabschnitts (6, 56) eine Versteifung vorgesehen ist.

5. Orthese nach einem der vorhergehenden Ansprüche, bei der zumindest ein Teilbereich des steifen Körpers (2, 52) zur individuellen Anpassung an die Hände verschiedener Benutzer plastisch verformbar ist.

6. Orthese nach einem der vorhergehenden Ansprüche, bei der das Element des steifen Körpers (2, 52) und/oder der Daumenabschnitt (6, 56) Metall, insbesondere Aluminium, und/oder Kunststoff aufweisen, insbesondere einen von einem Kunststoff umgebenen Metallkern, insbesondere einen Aluminiumkern.

7. Orthese nach Anspruch 2 oder nach Anspruch 2 und einem der Ansprüche 3 bis 6, bei der der Mittelhandabschnitt (5, 55) so dimensioniert ist, dass er sich über mindestens 30% des Umfangs der Ringöffnung (8, 58) erstreckt, die durch den dem Segment entsprechenden Ring definiert wird.

8. Orthese nach einem der vorhergehenden Ansprüche, bei der der steife Körper (2, 52) eine Polsterung aufweist, die so angeordnet ist, dass sie nach Befestigung an der Hand (20) eines Benutzers mit der Hand (20) in Anlage kommt.

9. Orthese nach Anspruch 1 bis 8, bei der der Daumenhalteabschnitt (107) ringsegmentförmig ausgebildet ist.

10. Orthese nach Anspruch 1 bis 9, bei der die Verschlusseinrichtung (170a, 170b) ferner mindestens einen zweiten Befestigungsabschnitt (170b) aufweist, der so angeordnet und ausgestaltet ist, dass er in dem angelegten Zustand der Orthese (101) in der Weise zumindest teilweise um den Daumen (22) geführt werden kann, dass er den Daumen (22) zusammen mit dem Daumenhalteabschnitt (107) ringförmig umschließt.

11. Orthese nach einem der Ansprüche 1 bis 10, bei der in jedem der beiden konkaven Abschnitte (151a, 151b) eine Durchgangsöffnung (153a, 153b) in der ersten Fläche und durch den Mittelhandabschnitt (105) ausgebildet ist.

12. Orthese nach einem der Ansprüche 1 bis 11, bei der der Mittelhandabschnitt (105) im Bereich des konvexen Abschnitts (152) in einer Richtung quer zur Richtung zwischen dem ersten (105a) und zweiten Ende (105b) des Mittelhandabschnitts (105) und entlang der ersten Fläche eine Verjüngung (154) aufweist.

13. Orthese nach einem der Ansprüche 1 bis 12, bei der der Mittelhandabschnitt (105) an seinem ersten Ende (105a) in einen flexiblen Abschnitt (150) übergeht.

## Claims

1. An orthosis for fastening to a human hand (20) for immobilizing the carpometacarpal joint and metacarpophalangeal joint of the thumb (22) of the hand (20), comprising a stiff body (2, 52, 102) with a metacarpal section (5, 55, 105) which is formed by a section of a curved and stiff element and which
- has a first end (5a, 55a, 105a) and a second end (5b, 55b, 105b), which are spaced apart from each other, and
- is designed in such a manner that said metacarpal section can be brought into engagement with a human hand (20) by being placed against the palm of the hand (20) below the metacarpophalangeal joints of an index finger, middle finger, ring finger and little finger such that it extends in a direction between the two edges of the hand over at least part of the palm and at least partially rests against the latter, and
a stiff thumb section (6, 56, 106) which
- extends from the metacarpal section (5, 55, 105) and has a first end (6a, 56a, 106a), which is fastened to the metacarpal section (5, 55, 105) or is formed by part of the metacarpal section (5, 55, 105), and a second end (6b, 56b, 106b),
- in the region of its second end (6b, 56b, 106b), has a ring-shaped or ring-segment-shaped thumb holding section (7, 57, 107) and
- is designed in such a manner that when the metacarpal section (5, 55, 105) is brought into engagement with the hand (20), the thumb section (6, 56, 106) can be placed at the thumb (22) of the hand (20) such that
- the thumb (22) is arranged in the ring opening (10, 60, 110) defined by the thumb holding section (7, 57, 107), and the thumb holding section (7, 57, 107) extends over at least part of the circumference of the thumb (22) and at least partially surrounds the latter and
- supports the thumb (22) against a movement in the direction of the palm in a position in which the thumb (22) and the index finger (23) of the hand (20) are put opposite each other and the index finger (23) can be moved toward the thumb (22) into contact therewith and can be moved away from the thumb (22),
wherein the metacarpal section (105) is designed in such a manner that, when the orthosis (101) is placed onto a human hand (20) and the metacarpal section (105) has been brought into engagement with the hand (20) and the thumb section (106) has been placed against the thumb (22) of the hand (20) such that the thumb (22) is arranged in the ring opening (110) defined by the thumb holding section (107) and the thumb holding section (107) extends over at least part of the circumference of the thumb (22) and at least partially surrounds the latter, said metacarpal section rests against the hand (20) exclusively in the region of the palm, and
wherein the orthosis furthermore has a closure device (170a, 170b) for fastening the orthosis (101) when placed on the hand (20), wherein the closure device (170a, 170b) has at least one first fastening section (170a) which is arranged and configured in such a manner that, when the orthosis (101) is placed on, said fastening section can be arranged and secured on the metacarpal section (105) and/or the thumb section (106) in such a manner that said fastening section runs over the back of the hand (20) and, together with the metacarpal section (105), forms at least part of an annularly closed section of the orthosis (51), which section surrounds the hand (20), and wherein the metacarpal section (105) has a first surface for contact with the palm and an opposite, second surface which, when the orthosis is put on, is directed away from the hand (20), wherein the first surface has two concave sections (151a, 151b) and a convex section (152) arranged between the latter, between the first end (105a) and the second end (105b) of the metacarpal section (105).

2. The orthosis as claimed in claim 1, in which
- the curved and stiff element and the section thereof which forms the metacarpal section (5, 55) are elongate,
- the first end (5a, 55a) of the metacarpal section (5, 55) is formed by one of the two longitudinal ends of the elongate element, and the metacarpal section (5, 55) has the first end (5a, 55a) and the second end (5b, 55b) in the longitudinal direction,
- the metacarpal section (5, 55) has the shape of a segment of a ring enclosing an elongate ring opening (8, 58), and therefore the first and the second end (5a, 55a; 5b, 55b) of the metacarpal section (5, 55) are spaced apart from each other,
- the metacarpal section (5, 55) is designed in such a manner that said metacarpal section can be brought into engagement with a human hand (20) by being placed around the back of the hand and the palm of the hand (20) below the metacarpophalangeal joints of an index finger, middle finger, ring finger and small finger such that it
- extends in a direction between the two edges of the hand over at least part of the back of the hand and at least partially rests against the latter,
- runs around at least one of the two edges of the hand and at least partially rests against the latter, and
- extends in a direction between the two edges of the hand over at least part of the palm and at least partially rests against the latter, and
the thumb section (6, 56) is elongate and has the first end (6a, 56a) and second end (6b, 56b) of the thumb section (6, 56) in the longitudinal direction thereof.

3. The orthosis as claimed in claim 1 or claim 2, in which the stiff body (2, 52) is formed from the element, and the thumb section (6, 56) is formed by a section of the element, wherein the thumb section (6, 56) extends from the second end (5b, 55b) of the metacarpal section (5, 55), and the first end (6a, 56a) of the thumb section (6, 56) is fastened to the second end (5b, 55b) of the metacarpal section (5, 55) or is formed by said end.

4. The orthosis as claimed in one of the preceding claims, in which a stiffener is provided in the region of the second end (5b, 55b) of the metacarpal section (5, 55) and/or of the first end (6a, 56a) of the thumb section (6, 56).

5. The orthosis as claimed in one of the preceding claims, in which at least a partial region of the stiff body (2, 52) is plastically deformable for individual adaptation to the hands of various users.

6. The orthosis as claimed in one of the preceding claims, in which the element of the stiff body (2, 52) and/or the thumb section (6, 56) comprise metal, in particular aluminum, and/or plastic, in particular a metal core surrounded by a plastic, in particular an aluminum core.

7. The orthosis as claimed in claim 2 or as claimed in claim 2 and one of claims 3 to 6, in which the metacarpal section (5, 55) is dimensioned in such a manner that said metacarpal section extends over at least 30% of the circumference of the ring opening (8, 58) which is defined by the ring corresponding to the segment.

8. The orthosis as claimed in one of the preceding claims, in which the stiff body (2, 52) has a padding which is arranged in such a manner that, following fastening to the hand (20) of a user, said padding comes into contact with the hand (20).

9. The orthosis as claimed in claim 1 to 8, in which the thumb holding section (107) is of ring-segment-shaped design.

10. The orthosis as claimed in claim 1 to 9, in which the closure device (170a, 170b) furthermore has at least one second fastening section (170b) which is arranged and configured such that, when the orthosis (101) is placed on, said fastening section can be at least partially guided around the thumb (22) in such a manner that said fastening section annularly surrounds the thumb (22) together with the thumb holding section (107).

11. The orthosis as claimed in one of claims 1 to 10, in which, in each of the two concave sections (151a, 151b), a passage opening (153a, 153b) is formed in the first surface and through the metacarpal section (105).

12. The orthosis as claimed in one of claims 1 to 11, in which, in the region of the convex section (152), the metacarpal section (105) has a tapered portion (154) in a direction transversely to the direction between the first end (105a) and the second end (105b) of the metacarpal section (105) and along the first surface.

13. The orthosis as claimed in one of claims 1 to 12, in which the metacarpal section (105) merges at its first end (105a) into a flexible section (150).

## Revendications

1. Orthèse destinée à être fixée à une main humaine (20) pour l'immobilisation de l'articulation trapézo-métacarpienne ou de l'articulation métacarpo-phalangienne du pouce (22) de la main (20) avec un corps rigide (2, 52, 102), avec
une portion centrale de main (5, 55, 105), qui est constituée d'une portion d'un élément incurvé et rigide et qui :
- comprend une première extrémité (5a, 55a, 105a) et une deuxième extrémité (5b, 55b, 105b) qui sont disposées à une certaine distance l'une de l'autre, et
- est conçu de façon à ce qu'il puisse être emboîté avec une main humaine (20) en l'appuyant en dessous des articulations métacarpo-phalangiennes de l'index, du majeur, de l'annulaire et du petit doigt, de façon à ce qu'il s'étende dans une direction entre la tranche de la main (20) sur au moins une partie de la paume de la main et s'appuie au moins partiellement contre celle-ci, et
une portion de pouce rigide (6, 56, 106) qui :
- s'étend de la portion centrale de la main (5, 55, 105) et comprend une première extrémité (6a, 56a, 106a), qui est fixée à la portion centrale de la main (5, 55, 105) ou est constituée d'une partie de la portion centrale de la main (5, 55, 105), et une deuxième extrémité (6b, 56b, 106b),
- comprend, au niveau de sa deuxième extrémité (6b, 56b, 106b), une portion de maintien du pouce (7, 57, 107) de forme annulaire ou en forme de segment annulaire, et
- est conçu de façon à ce que la portion de pouce (6, 56, 106) peut être appuyée contre le pouce (22) de la main (20) lorsque la portion centrale de la main (5, 55, 105) est emboîtée avec la main (20) de façon à ce que
- le pouce (22) est disposé dans l'ouverture annulaire (10, 60, 110) définie par la portion de maintien du pouce (7, 57, 107) et la portion de maintien du pouce (7, 57, 107) s'étend sur au moins une partie de la circonférence du pouce (22) et l'entoure au moins partiellement et
- soutient le pouce (22) afin d'empêcher un mouvement en direction de la paume de la main dans une position dans laquelle le pouce (22) et l'index (23) de la main (20) sont opposés et l'index (23) peut être déplacé en direction du pouce (22) en appui contre celui-ci et éloigné du pouce (22),
la portion centrale de la main (105) étant conçue de façon à ce que, dans l'état de l'orthèse (101) appuyé contre une main humaine (20), dans lequel la portion centrale de la main (105) a été emboîtée avec la main (20) et la portion de pouce (106) a été appuyée contre le pouce (22) de la main (20) de façon à ce que le pouce (22) soit disposé dans l'ouverture annulaire (110) définie par la portion de maintien du pouce (107) et la portion de maintien du pouce (107) s'étende sur au moins une partie de la circonférence du pouce (22) et l'entoure au moins partiellement, elle s'appuie exclusivement au niveau de la paume de la main (20), et
l'orthèse comprenant en outre un dispositif de fermeture (107a, 107b) pour la fixation de l'orthèse (101) dans l'état appuyé contre la main (20), le dispositif de fermeture (107a, 107b) comprenant au moins une première portion de fixation (170a), qui est disposée et conçue de façon à ce que, dans l'état appuyé de l'orthèse (101), elle puisse être disposée et fixée à la portion centrale de la main (105) et/ou à la portion du pouce (106), de façon à ce qu'elle s'étende sur le dos de la main (20) et constitue, avec la portion centrale de la main (105), au moins une partie d'une portion fermée annulaire de l'orthèse (51), qui entoure la main (20), et
la portion centrale de la main (105) comprenant une première surface pour l'appui contre la paume de la main et une deuxième surface opposée, qui est orientée loin de la main (20) dans l'état appuyé de l'orthèse, la première surface comprenant, entre la première (105a) et la deuxième extrémité (105b) de la portion centrale de la main (105), deux portions concaves (151a, 151b) et une portion convexe (152) située entre elles.

2. Orthèse selon la revendication 1, dans laquelle
- l'élément incurvé et rigide et sa portion qui constitue la portion centrale de la main (5, 55), sont oblongs,
- la première extrémité (5a, 55a) de la portion centrale de la main (5, 55) est constituée par une des deux extrémités longitudinales de l'élément oblong et la portion centrale de la main (5, 55) comprend la première extrémité (5a, 55a) et la deuxième extrémité (5b, 55b) dans la direction longitudinale,
- la portion centrale de la main (5, 55) présente la forme d'un segment d'un anneau entourant une ouverture annulaire oblongue (8, 58), de façon à ce que la première et la deuxième extrémité (5a, 55a ; 5b, 55b) de la portion centrale de la main (5, 55) soient disposées à une certaine distance entre elles,
- la portion centrale de la main (5, 55) est conçue de façon à ce qu'elle puisse être emboîtée avec une main humaine (20), en la posant en dessous des articulations métacarpo-phalangiennes de l'index, du majeur, de l'annulaire et du petit doigt, autour du dos de la main et de la paume de la main (20), de façon à ce que
- elle s'étende dans une direction entre des deux tranches de la main sur au moins une partie du dos de la main et s'appuie au moins partiellement contre celui-ci
- elle s'étende autour d'au moins une des deux tranches de la main et s'appuie au moins partiellement contre celle-ci et
- elle s'étende dans une direction entre les deux tranches de la main sur au moins une partie de la paume de la main et s'appuie au moins partiellement contre celle-ci et
la portion de pouce (6, 56) est oblongue et comprend la première extrémité (6a, 56a) et la deuxième extrémité (6b, 56b) de la portion de pouce (6, 56) dans sa direction longitudinale.

3. Orthèse selon la revendication 1 ou la revendication 2, dans laquelle le corps rigide (2, 52) est constitué de l'élément et la portion de pouce (6, 56) est constitué d'une portion de l'élément, la portion de pouce (6, 56) s'étendant à partir de la deuxième extrémité (5b, 55b) de la portion centrale de la main (5, 55) et la première extrémité (6a, 56a) de la portion de pouce (6, 56) étant fixée à la deuxième extrémité (5b, 55b) de la portion centrale de la main (5, 55) ou étant constituée de celle-ci.

4. Orthèse selon l'une des revendications précédentes, dans laquelle, au niveau de la deuxième extrémité (5b, 55b) de la portion centrale de la main (5, 55) et/ou de la première extrémité (6a, 56a) de la portion de pouce (6, 56), est prévue une rigidification.

5. Orthèse selon l'une des revendications précédentes, dans laquelle au moins une partie du corps rigide (2, 52) peut être déformée de manière plastique pour une adaptation individuelle aux mains de différents utilisateurs.

6. Orthèse selon l'une des revendications précédentes, dans laquelle l'élément du corps rigide (2, 52) et/ou la portion de pouce (6, 56) comprennent du métal, plus particulièrement de l'aluminium, et/ou une matière plastique, plus particulièrement un noyau métallique entouré d'une matière plastique, plus particulièrement un noyau en aluminium.

7. Orthèse selon la revendication 2 ou selon la revendication 2 et une des revendications 3 à 6, dans laquelle la portion centrale de la main (5, 55) est dimensionnée de façon à ce qu'elle s'étende sur au moins 30 % de la circonférence de l'ouverture annulaire (8, 58), qui est définie par l'anneau correspondant au segment.

8. Orthèse selon l'une des revendications précédentes, dans laquelle le corps rigide (2, 52) présente un rembourrage qui est disposé de façon à ce que, après la fixation, il soit en appui contre la main (20) d'un utilisateur avec la main (20).

9. Orthèse selon l'une des revendications 1 à 8, dans laquelle la portion de maintien du pouce (107) présente la forme d'un segment d'anneau.

10. Orthèse selon l'une des revendications 1 à 9, dans laquelle le dispositif de fermeture (170a, 170b) comprend en outre au moins une deuxième portion de fixation (170b) qui est disposée et conçue de façon à ce que, dans l'état appuyé de l'orthèse (101), elle puisse être guidée au moins partiellement autour du pouce (22) de façon à ce qu'elle entoure de manière annulaire le pouce (22) ainsi que la portion de maintien du pouce (107).

11. Orthèse selon l'une des revendications 1 à 10, dans laquelle, dans chacune de deux portions concaves (151 a, 151b), une ouverture de passage (153a, 153b) est réalisée dans la première surface et à travers la portion centrale de la main (105).

12. Orthèse selon l'une des revendications 1 à 11, dans laquelle la portion centrale de la main (105) comprend, au niveau de la portion convexe (152), dans une direction transversale par rapport à la direction, entre la première extrémité (105a) et la deuxième extrémité (105b) de la portion centrale de la main (105) et le long de la première surface, un rétrécissement (154).

13. Orthèse selon l'une des revendications 1 à 12, dans laquelle la portion centrale de la main (105) est prolongée, au niveau de sa première extrémité (105a), par une portion flexible (150).
